# EUROPEAN PATENT SPECIFICATION

(11) **EP 1 169 434 B1**
(45) Date of publication and mention of the grant of the patent: **11.02.2009**
(21) Application number: 00912414.0
(22) Date of filing: 28.03.2000
(51) Int. Cl.: C12N 9/28, C11D 3/386

(54) **POLYPEPTIDES HAVING ALKALINE ALPHA-AMYLASE ACTIVITY AND NUCLEIC ACIDS ENCODING SAME**
POLYPEPTIDE MIT ALKALINER ALPHA-AMYLASE-AKTIVITÄT UND FÜR DIESE KODIERENDE NUKLEINSÄUREN
POLYPEPTIDES POSSEDANT UNE ACTIVITE ALCALINE ALPHA-AMYLASE ET ACIDES NUCLEIQUES CODANT POUR CES POLYPEPTIDES

(30) Priority: 31.03.1999 DK 99438; 13.04.1999 DK 99489
(43) Date of publication of application: 09.01.2002
(73) Proprietor: Novozymes A/S, 2880 Bagsvaerd (DK)
(72) Inventor: OUTTRUP, Helle, DK-3500 Vaerloese (DK); HOECK, Lisbeth Hedegaard, DK- 5871 Froerup (DK); NIELSEN, Bjarne, Roenfeldt, DK-2830 Virum (DK); BORCHERT, Torben Vedel, DK-3460 Birkeroed (DK); NIELSEN, Vibeke, Skovgaard, DK-2880 Bagsvaerd (DK); BISGARD-FRANTZEN, Henrik, DK-2880 Bagsvaerd (DK); SVENDSEN, Allan, DK-3460 Birkeroed (DK); ANDERSEN, Carsten, DK-3500 Vaerloese (DK)
(86) International application number: PCT/DK2000/000147
(87) International publication number: WO 2000/060058

(56) References cited:
- WO-A-96/23873
- WO-A-97/32961
- IGARASHI K ET AL: "Improved Thermostability of a Bacillus alpha-Amylase by Deletion of an Arginine-Glycine Residue Is Caused by Enhanced Calcium Binding" BIOCHEM BIOPHYS RES COMMUN, vol. 248, no. 2, 1998, pages 372-377, XP002901159
- TSUKAMOTO A ET AL: "Nucleotide Sequence of the Maltohexaose-Producing Amylase Gene from an Alkalophilic Bacillus sp. #707 and Structural Similarity to Liquefying Type Alpha-Amylases" BIOCHEMICAL AND BIOPHYSICAL RESEARCH COMMUNICATIONS, vol. 151, no. 1, 29 February 1988 (1988-02-29), pages 25-31, XP002901160

### SEQUENCE LISTING

<110> Novo Nordisk A/S
<120> POLYPEPTIDES HAVING ALKALINE ALPHA-AMYLASE ACTIVITY AND NUCLEIC ACIDS ENCODING SAME
<160> 12
<170> FastSEQ for Windows Version 3.0
<210> 1
   <211> 1458
   <212> DNA
   <213> Bacillus sp.
<220>
   <221> CDS
   <222> (1)...(1458)
<221> mat_peptide
   <222> (1)...(1458)
<400> 1
   cat cac gat ggg acg aac gga acg att atg caa tat ttt gaa tgg aac      48
   His His Asp Gly Thr Asn Gly Thr Ile Met Gln Tyr Phe Glu Trp Asn
    1               5                  10                  15
   gtt ccg aat gat gga caa cat tgg aac cgc tta cac aac aat gct cag      96
   Val Pro Asn Asp Gly Gln His Trp Asn Arg Leu His Asn Asn Ala Gln
               20                  25                  30
   aat tta aaa aat gct gga att act gcg att tgg att ccc cct gca tgg     144
   Asn Leu Lys Asn Ala Gly Ile Thr Ala Ile Trp Ile Pro Pro Ala Trp
           35                  40                  45
   aaa gga acg agc caa aat gat gta ggc tat ggt gcg tat gac ctc tat     192
   Lys Gly Thr Ser Gln Asn Asp Val Gly Tyr Gly Ala Tyr Asp Leu Tyr
       50                  55                  60
   gac ctt ggt gaa ttt aat caa aaa gga aca gta cgt acg aaa tat ggc     240
   Asp Leu Gly Glu Phe Asn Gln Lys Gly Thr Val Arg Thr Lys Tyr Gly
    65                  70                  75                  80
   aca aaa gca gaa tta gaa cga gcg att cgc tcg tta aaa gcg aac ggg     288
   Thr Lys Ala Glu Leu Glu Arg Ala Ile Arg Ser Leu Lys Ala Asn Gly
                   85                  90                  95
   att caa gtg tac ggt gat gtc gtc atg aac cat aaa ggt ggt gct gat     336
   Ile Gln Val Tyr Gly Asp Val Val Met Asn His Lys Gly Gly Ala Asp
               100                 105                 110
   ttt acc gag cga gtt caa gcg gtt gaa gta aat ccg caa aat cga aat     384
   Phe Thr Glu Arg Val Gln Ala Val Glu Val Asn Pro Gln Asn Arg Asn
           115                 120                 125
   caa gaa gtg tct ggc act tat gaa atc gaa gca tgg act gga ttt aat      432
   Gln Glu Val Ser Gly Thr Tyr Glu Ile Glu Ala Trp Thr Gly Phe Asn
       130                 135                 140
   ttc cct gga cgt ggc aat caa cac tct tcg ttt aaa tgg cgt tgg tac      480
   Phe Pro Gly Arg Gly Asn Gln His Ser Ser Phe Lys Trp Arg Trp Tyr
   145                 150                 155                 160
   cat ttt gat gga acg gac tgg gat caa tct cgt caa ctc tca aat cgt      528
   His Phe Asp Gly Thr Asp Trp Asp Gln Ser Arg Gln Leu Ser Asn Arg
                   165                 170                 175
   atc tat aaa ttt aga gga gac ggg aaa gca tgg gac tgg gaa gta gac      576
   Ile Tyr Lys Phe Arg Gly Asp Gly Lys Ala Trp Asp Trp Glu Val Asp
               180                 185                 190
   act gaa aac gga aac tac gat tac tta atg tat gca gac gtt gac atg      624
   Thr Glu Asn Gly Asn Tyr Asp Tyr Leu Met Tyr Ala Asp Val Asp Met
           195                 200                 205
   aac cat cct gaa gtc ata aac gaa tta aac cga tgg ggc gta tgg tac      672
   Asn His Pro Glu Val Ile Asn Glu Leu Asn Arg Trp Gly Val Trp Tyr
       210                 215                 220
   gca aat acc ctt aat tta gac ggc ttt aga ctt gat gcg gtg aag cat      720
   Ala Asn Thr Leu Asn Leu Asp Gly Phe Arg Leu Asp Ala Val Lys His
   225                 230                 235                 240
   att caa ttc agc ttc atg cgc aac tgg ctc gga cat gtt cga gga caa      768
   Ile Gln Phe Ser Phe Met Arg Asn Trp Leu Gly His Val Arg Gly Gln
                   245                 250                 255
   aca ggc aag aac ctc ttt gca gtt gca gaa tat tgg aaa aac gac tta      816
   Thr Gly Lys Asn Leu Phe Ala Val Ala Glu Tyr Trp Lys Asn Asp Leu
               260                 265                 270
   ggt gcc cta gaa aat tat tta agt aaa aca aat tgg acg atg agc gcc      864
   Gly Ala Leu Glu Asn Tyr Leu Ser Lys Thr Asn Trp Thr Met Ser Ala
           275                 280                 285
   ttt gat gtg cct ctt cat tac aac ctt tac caa gcg tca aat agc ggc      912
   Phe Asp Val Pro Leu His Tyr Asn Leu Tyr Gln Ala Ser Asn Ser Gly
       290                 295                 300
   gga aat tac gac atg aga aat ttg tta aat gga acg ctc gtt cag cgt      960
   Gly Asn Tyr Asp Met Arg Asn Leu Leu Asn Gly Thr Leu Val Gln Arg
   305                 310                 315                 320
   cac cct agt cat gca gtg acg ttc gtc gat aac cat gac aca cag cct     1008
   His Pro Ser His Ala Val Thr Phe Val Asp Asn His Asp Thr Gln Pro
                   325                 330                 335
   gga gaa gct ctt gag tcg ttc gtc caa ggc tgg ttt aaa ccg ctc gct     1056
   Gly Glu Ala Leu Glu Ser Phe Val Gln Gly Trp Phe Lys Pro Leu Ala
           340                 345                 350
   tat gca acg att ctt acg aga gaa caa ggc tac cca caa gtg ttt tac      1104
   Tyr Ala Thr Ile Leu Thr Arg Glu Gln Gly Tyr Pro Gln Val Phe Tyr
       355                 360                 365
   ggc gat tat tat ggt att ccg agt gat ggc gtt cca agc tat cgt caa      1152
   Gly Asp Tyr Tyr Gly Ile Pro Ser Asp Gly Val Pro Ser Tyr Arg Gln
   370                 375                 380
   caa att gat cca ctt tta aaa gcc cgt cag caa tat gct tat gga aga      1200
   Gln Ile Asp Pro Leu Leu Lys Ala Arg Gln Gln Tyr Ala Tyr Gly Arg
   385                 390                 395                 400
   cag cac gat tac ttc gat cat tgg gat gta att ggc tgg acg cgt gaa      1248
   Gln His Asp Tyr Phe Asp His Trp Asp Val Ile Gly Trp Thr Arg Glu
                   405                 410                 415
   ggg aac gcg tct cac ccg aat tct ggg ctt gcg acc ata atg tcc gat      1296
   Gly Asn Ala Ser His Pro Asn Ser Gly Leu Ala Thr Ile Met Ser Asp
               420                 425                 430
   ggt cca ggt gga tcg aaa tgg atg tat gtt ggt cgt caa aaa gct ggt      1344
   Gly Pro Gly Gly Ser Lys Trp Met Tyr Val Gly Arg Gln Lys Ala Gly
           435                 440                 445
   gaa gta tgg cat gac ata act gga aac cgc agc ggc act gtg aca att      1392
   Glu Val Trp His Asp Ile Thr Gly Asn Arg Ser Gly Thr Val Thr Ile
       450                 455                 460
   aac cag gac ggc tgg gga caa ttt ttt gtc aac ggt ggt tcc gtc tcc      1440
   Asn Gln Asp Gly Trp Gly Gln Phe Phe Val Asn Gly Gly Ser Val Ser
   465                 470                 475                 480
   gta tgg gtg aaa cga taa                                              1458
   Val Trp Val Lys Arg  *
                   485
<210> 2
   <211> 485
   <212> PRT
   <213> Bacillus sp.
<400> 2
   His His Asp Gly Thr Asn Gly Thr Ile Met Gln Tyr Phe Glu Trp Asn
   1               5                   10                  15
   Val Pro Asn Asp Gly Gln His Trp Asn Arg Leu His Asn Asn Ala Gln
               20                  25                  30
   Asn Leu Lys Asn Ala Gly Ile Thr Ala Ile Trp Ile Pro Pro Ala Trp
           35                  40                  45
   Lys Gly Thr Ser Gln Asn Asp Val Gly Tyr Gly Ala Tyr Asp Leu Tyr
       50                  55                  60
   Asp Leu Gly Glu Phe Asn Gln Lys Gly Thr Val Arg Thr Lys Tyr Gly
   65                  70                  75                  80
   Thr Lys Ala Glu Leu Glu Arg Ala Ile Arg Ser Leu Lys Ala Asn Gly
   85 90 95
   Ile Gln Val Tyr Gly Asp Val Val Met Asn His Lys Gly Gly Ala Asp
   100 105 110
   Phe Thr Glu Arg Val Gln Ala Val Glu Val Asn Pro Gln Asn Arg Asn
   115 120 125
   Gln Glu Val Ser Gly Thr Tyr Glu Ile Glu Ala Trp Thr Gly Phe Asn
   130 135 140
   Phe Pro Gly Arg Gly Asn Gln His Ser Ser Phe Lys Trp Arg Trp Tyr
   145 150 155 160
   His Phe Asp Gly Thr Asp Trp Asp Gln Ser Arg Gln Leu Ser Asn Arg
   165 170 175
   Ile Tyr Lys Phe Arg Gly Asp Gly Lys Ala Trp Asp Trp Glu Val Asp
   180 185 190
   Thr Glu Asn Gly Asn Tyr Asp Tyr Leu Met Tyr Ala Asp Val Asp Met
   195 200 205
   Asn His Pro Glu Val Ile Asn Glu Leu Asn Arg Trp Gly Val Trp Tyr
   210 215 220
   Ala Asn Thr Leu Asn Leu Asp Gly Phe Arg Leu Asp Ala Val Lys His
   225 230 235 240
   Ile Gln Phe Ser Phe Met Arg Asn Trp Leu Gly His Val Arg Gly Gln
   245 250 255
   Thr Gly Lys Asn Leu Phe Ala Val Ala Glu Tyr Trp Lys Asn Asp Leu
   260 265 270
   Gly Ala Leu Glu Asn Tyr Leu Ser Lys Thr Asn Trp Thr Met Ser Ala
   275 280 285
   Phe Asp Val Pro Leu His Tyr Asn Leu Tyr Gln Ala Ser Asn Ser Gly
   290 295 300
   Gly Asn Tyr Asp Met Arg Asn Leu Leu Asn Gly Thr Leu Val Gln Arg
   305 310 315 320
   His Pro Ser His Ala Val Thr Phe Val Asp Asn His Asp Thr Gln Pro
   325 330 335
   Gly Glu Ala Leu Glu Ser Phe Val Gln Gly Trp Phe Lys Pro Leu Ala
   340 345 350
   Tyr Ala Thr Ile Leu Thr Arg Glu Gln Gly Tyr Pro Gln Val Phe Tyr
   355 360 365
   Gly Asp Tyr Tyr Gly Ile Pro Ser Asp Gly Val Pro Ser Tyr Arg Gln
   370 375 380
   Gln Ile Asp Pro Leu Leu Lys Ala Arg Gln Gln Tyr Ala Tyr Gly Arg
   385 390 395 400
   Gln His Asp Tyr Phe Asp His Trp Asp Val Ile Gly Trp Thr Arg Glu
   405 410 415
   Gly Asn Ala Ser His Pro Asn Ser Gly Leu Ala Thr Ile Met Ser Asp
   420 425 430
   Gly Pro Gly Gly Ser Lys Trp Met Tyr Val Gly Arg Gln Lys Ala Gly
   435 440 445
   Glu Val Trp His Asp Ile Thr Gly Asn Arg Ser Gly Thr Val Thr Ile
   450 455 460
   Asn Gln Asp Gly Trp Gly Gln Phe Phe Val Asn Gly Gly Ser Val Ser
   465 470 475 480
   Val Trp Val Lys Arg
   485
<210> 3
   <211> 1458
   <212> DNA
   <213> Bacillus sp.
<220>
   <221> CDS
   <222> (1)...(1458)
<221> mat_peptide
   <222> (1)...(1458)
<400> 3
   cat cac gat ggg acg aac gga acg att atg cag tat ttt gaa tgg aac       48
   His His Asp Gly Thr Asn Gly Thr Ile Met Gln Tyr Phe Glu Trp Asn
    1               5                  10                  15
   gtt ccg aat gat gga caa cat tgg aac cgc tta cac aac aac gct caa       96
   Val Pro Asn Asp Gly Gln His Trp Asn Arg Leu His Asn Asn Ala Gln
               20                  25                  30
   aat tta aaa aat gcc gga att aca gca atc tgg att cca cct gcg tgg      144
   Asn Leu Lys Asn Ala Gly Ile Thr Ala Ile Trp Ile Pro Pro Ala Trp
           35                  40                  45
   aaa gga acg agc caa aat gat gta ggc tac ggt gcg tat gac ctt tat      192
   Lys Gly Thr Ser Gln Asn Asp Val Gly Tyr Gly Ala Tyr Asp Leu Tyr
       50                  55                  60
   gac ctt ggt gaa ttt aac caa aaa gga acg gtc cgt acg aaa tat gga      240
   Asp Leu Gly Glu Phe Asn Gln Lys Gly Thr Val Arg Thr Lys Tyr Gly
    65                  70                  75                  80
   aca aaa gca gaa tta gaa cga gcg att cgt tcg tta aag gcg aac ggg      288
   Thr Lys Ala Glu Leu Glu Arg Ala Ile Arg Ser Leu Lys Ala Asn Gly
                   85                  90                  95
   att caa gtg tat ggc gat gtt gtt atg aac cat aaa ggc gga gct gat      336
   Ile Gln Val Tyr Gly Asp Val Val Met Asn His Lys Gly Gly Ala Asp
               100                 105                 110
   ttc acc gag cgt gtt caa gcg gtt gaa gtg aac ccg caa aac cga aac      384
   Phe Thr Glu Arg Val Gln Ala Val Glu Val Asn Pro Gln Asn Arg Asn
           115                 120                 125
   caa gaa gtg tct ggc act tat caa atc gaa gca tgg aca ggg ttc aat      432
   Gln Glu Val Ser Gly Thr Tyr Gln Ile Glu Ala Trp Thr Gly Phe Asn
       130                 135                 140
   ttt cct gga cgt ggc aat caa cat tct tcg ttt aaa tgg cgc tgg tat      480
   Phe Pro Gly Arg Gly Asn Gln His Ser Ser Phe Lys Trp Arg Trp Tyr
   145                 150                 155                 160
   cat ttc gat ggg acg gat tgg gac cag tct cgc caa ctc gca aat cgt      528
   His Phe Asp Gly Thr Asp Trp Asp Gln Ser Arg Gln Leu Ala Asn Arg
                   165                 170                 175
   att tat aag ttt aga gga gac gga aaa gca tgg gac tgg gaa gtt gac      576
   Ile Tyr Lys Phe Arg Gly Asp Gly Lys Ala Trp Asp Trp Glu Val Asp
                   180                      185                      190
   act gaa aat ggg aac tat gat tac tta atg tat gca gac gtt gac atg        624
   Thr Glu Asn Gly Asn Tyr Asp Tyr Leu Met Tyr Ala Asp Val Asp Met
           195                     200                     205
   gat cat cca gaa gtg att aac gaa cta aac cgt tgg ggc gtc tgg tac        672
   Asp His Pro Glu Val Ile Asn Glu Leu Asn Arg Trp Gly Val Trp Tyr
       210                     215                     220
   gcg aat acc ctt aat tta gac ggc ttc cga ctg gat gca gtg aaa cat        720
   Ala Asn Thr Leu Asn Leu Asp Gly Phe Arg Leu Asp Ala Val Lys His
   225                     230                     235                     240
   att aaa ttt agc ttc atg cgt gat tgg tta ggg cat gtt cgc ggg caa        768
   Ile Lys Phe Ser Phe Met Arg Asp Trp Leu Gly His Val Arg Gly Gln
                   245                     250                     255
   acg ggc aag aat ctt ttt gcc gtt gca gag tat tgg aag aat gac cta        816
   Thr Gly Lys Asn Leu Phe Ala Val Ala Glu Tyr Trp Lys Asn Asp Leu
               260                     265                     270
   ggg gct tta gaa aat tat tta agc aaa aca aat tgg acg atg agc gcc        864
   Gly Ala Leu Glu Asn Tyr Leu Ser Lys Thr Asn Trp Thr Met Ser Ala
           275                     280                     285
   ttt gat gtt ccg ctt cat tac aac ctt tat caa gcg tca aat agt agc        912
   Phe Asp Val Pro Leu His Tyr Asn Leu Tyr Gln Ala Ser Asn Ser Ser
       290                     295                     300
   gga aat tac gac atg aga aac ttg tta aat gga aca ctc gtt caa cgt        960
   Gly Asn Tyr Asp Met Arg Asn Leu Leu Asn Gly Thr Leu Val Gln Arg
   305                     310                     315                     320
   cat ccg agc cat gcg gtt acg ttt gtc gat aac cac gac aca cag cct       1008
   His Pro Ser His Ala Val Thr Phe Val Asp Asn His Asp Thr Gln Pro
                   325                     330                     335
   gga gaa gcc ctc gaa tcg ttc gtt caa ggc tgg ttt aaa cca cta gct       1056
   Gly Glu Ala Leu Glu Ser Phe Val Gln Gly Trp Phe Lys Pro Leu Ala
               340                     345                     350
   tat gca acg att ctt acg aga gag caa ggc tac cca caa gtg ttt tac       1104
   Tyr Ala Thr Ile Leu Thr Arg Glu Gln Gly Tyr Pro Gln Val Phe Tyr
           355                     360                     365
   ggc gat tat tat ggc atc cca agt gac ggt gtt cca agc tac cgt caa       1152
   Gly Asp Tyr Tyr Gly Ile Pro Ser Asp Gly Val Pro Ser Tyr Arg Gln
       370                     375                     380
   cag atc gac cca ctt tta aaa gct cgt caa caa tat gct tat ggt aga       1200
   Gln Ile Asp Pro Leu Leu Lys Ala Arg Gln Gln Tyr Ala Tyr Gly Arg
   385                     390                     395                     400
   cag cac gat tac ttt gat cat tgg gat gta att ggc tgg aca cgt gaa       1248
   Gln His Asp Tyr Phe Asp His Trp Asp Val Ile Gly Trp Thr Arg Glu
                       405                 410                 415
   gga aac gca tct cac ccg aac tca gga ctt gca acc att atg tct gat      1296
   Gly Asn Ala Ser His Pro Asn Ser Gly Leu Ala Thr Ile Met Ser Asp
                   420                 425                 430
   ggt cca ggt gga tca aaa tgg atg tat gtt ggc cgt cag aaa gct ggc      1344
   Gly Pro Gly Gly Ser Lys Trp Met Tyr Val Gly Arg Gln Lys Ala Gly
               435                 440                 445
   gaa gtg tgg cat gac atg act gga aac cgc agt ggc act gtg aca att      1392
   Glu Val Trp His Asp Met Thr Gly Asn Arg Ser Gly Thr Val Thr Ile
           450                 455                 460
   aat caa gac ggc tgg gga cac ttt ttt gtc aac ggc ggc tct gtc tcc      1440
   Asn Gln Asp Gly Trp Gly His Phe Phe Val Asn Gly Gly Ser Val Ser
   465                 470                 475                 480
   gta tgg gtg aaa cga taa                                              1458
   Val Trp Val Lys Arg  *
                   485
<210> 4
   <211> 485
   <212> PRT
   <213> Bacillus sp.
<400> 4
   His His Asp Gly Thr Asn Gly Thr Ile Met Gln Tyr Phe Glu Trp Asn
    1               5                  10                  15
   Val Pro Asn Asp Gly Gln His Trp Asn Arg Leu His Asn Asn Ala Gln
               20                  25                  30
   Asn Leu Lys Asn Ala Gly Ile Thr Ala Ile Trp Ile Pro Pro Ala Trp
           35                  40                  45
   Lys Gly Thr Ser Gln Asn Asp Val Gly Tyr Gly Ala Tyr Asp Leu Tyr
       50                  55                  60
   Asp Leu Gly Glu Phe Asn Gln Lys Gly Thr Val Arg Thr Lys Tyr Gly
   65                  70                  75                  80
   Thr Lys Ala Glu Leu Glu Arg Ala Ile Arg Ser Leu Lys Ala Asn Gly
                   85                  90                  95
   Ile Gln Val Tyr Gly Asp Val Val Met Asn His Lys Gly Gly Ala Asp
               100                 105                 110
   Phe Thr Glu Arg Val Gln Ala Val Glu Val Asn Pro Gln Asn Arg Asn
           115                 120                 125
   Gln Glu Val Ser Gly Thr Tyr Gln Ile Glu Ala Trp Thr Gly Phe Asn
       130                 135                 140
   Phe Pro Gly Arg Gly Asn Gln His Ser Ser Phe Lys Trp Arg Trp Tyr
   145                 150                 155                 160
   His Phe Asp Gly Thr Asp Trp Asp Gln Ser Arg Gln Leu Ala Asn Arg
                   165                 170                 175
   Ile Tyr Lys Phe Arg Gly Asp Gly Lys Ala Trp Asp Trp Glu Val Asp
               180                 185                 190
   Thr Glu Asn Gly Asn Tyr Asp Tyr Leu Met Tyr Ala Asp Val Asp Met
           195                 200                 205
   Asp His Pro Glu Val Ile Asn Glu Leu Asn Arg Trp Gly Val Trp Tyr
           210                     215                     220
   Ala Asn Thr Leu Asn Leu Asp Gly Phe Arg Leu Asp Ala Val Lys His
   225                 230                     235                 240
   Ile Lys Phe Ser Phe Met Arg Asp Trp Leu Gly His Val Arg Gly Gln
                   245                     250                     255
   Thr Gly Lys Asn Leu Phe Ala Val Ala Glu Tyr Trp Lys Asn Asp Leu
               260                     265                     270
   Gly Ala Leu Glu Asn Tyr Leu Ser Lys Thr Asn Trp Thr Met Ser Ala
           275                     280                     285
   Phe Asp Val Pro Leu His Tyr Asn Leu Tyr Gln Ala Ser Asn Ser Ser
       290                     295                     300
   Gly Asn Tyr Asp Met Arg Asn Leu Leu Asn Gly Thr Leu Val Gln Arg
   305                 310                     315                 320
   His Pro Ser His Ala Val Thr Phe Val Asp Asn His Asp Thr Gln Pro
                   325                     330                     335
   Gly Glu Ala Leu Glu Ser Phe Val Gln Gly Trp Phe Lys Pro Leu Ala
               340                     345                     350
   Tyr Ala Thr Ile Leu Thr Arg Glu Gln Gly Tyr Pro Gln Val Phe Tyr
           355                     360                     365
   Gly Asp Tyr Tyr Gly Ile Pro Ser Asp Gly Val Pro Ser Tyr Arg Gln
       370                     375                     380
   Gln Ile Asp Pro Leu Leu Lys Ala Arg Gln Gln Tyr Ala Tyr Gly Arg
   385                 390                     395                 400
   Gln His Asp Tyr Phe Asp His Trp Asp Val Ile Gly Trp Thr Arg Glu
                   405                     410                     415
   Gly Asn Ala Ser His Pro Asn Ser Gly Leu Ala Thr Ile Met Ser Asp
               420                     425                     430
   Gly Pro Gly Gly Ser Lys Trp Met Tyr Val Gly Arg Gln Lys Ala Gly
           435                     440                     445
   Glu Val Trp His Asp Met Thr Gly Asn Arg Ser Gly Thr Val Thr Ile
       450                     455                     460
   Asn Gln Asp Gly Trp Gly His Phe Phe Val Asn Gly Gly Ser Val Ser
   465                 470                     475                 480
   Val Trp Val Lys Arg
                   485
<210> 5
   <211> 7
   <212> PRT
   <213> unknown
<220>
   <223> Xaa=Leu, Val, Ile
<223> Xaa=Ile, Leu
<223> Degenerate Primer Region
<400> 5
   Gly Ile Thr Ala Xaa Trp Xaa
   1               5
<210> 6
   <211> 9
   <212> PRT
   <213> unknown
<220>
   <223> degenerate primer region
<223> Xaa=Gly, Ala
<223> Xaa=Val, Phe, Leu
<223> Xaa=Met, Leu, Ile, Phe
<400> 6
   Val Tyr Xaa Asp Xaa Val Xaa Asn His
   1 5
<210> 7
   <211> 10
   <212> PRT
   <213> unknown
<220>
   <223> Xaa=Phe, Ile
<223> Xaa=Phe, Leu, Ile, Val
<223> Xaa=Ala, Val
<223> Degenerate Primer Region
<400> 7
   Asp Gly Xaa Arg Xaa Asp Ala Xaa Lys His
   1 5 10
<210> 8
   <211> 10
   <212> PRT
   <213> unkwnown
<220>
   <223> Degenerate Primer Region
<223> Xaa=Phe, Ile
<223> Xaa=Phe, Leu, Ile, Val
<223> Xaa=Ala, Val
<400> 8
   Asp Gly Xaa Arg Xaa Asp Ala Xaa Lys His
   1 5 10
<210> 9
   <211> 8
   <212> PRT
   <213> unknown
<220>
   <223> Degenerate Primer Region
<223> Xaa=Asp, Glu
<400> 9
   Val Thr Phe Val Xaa Asn His Asp
   1               5
<210> 10
   <211> 6
   <212> PRT
   <213> unknown
<220>
   <223> Degenerate Primer Region
<400> 10
   Gly Trp Thr Arg Glu Gly
   1               5
<210> 11
   <211> 47
   <212> DNA
   <213> Artificial Sequence
<220>
   <223> Primer 197cloningC
<400> 11
   ccgagctcgg ccggctgggc cgtcgactta tcgtttcacc catacgg 47
<210> 12
   <211> 38
   <212> DNA
   <213> Artificial Sequence
<220>
   <223> 197cloningN
<400> 12
   cattctgcag cagcggcgca tcacgatggg acgaacgg 38

## Description

### Background of the Invention

### Field of the Invention

The present invention relates to isolated polypeptides having α-amylase activity and isolated nucleic acid sequences encoding the polypeptides. The invention also relates to nucleic acid constructs, vectors, and host cells comprising the nucleic acid sequences as well as methods for producing and using the polypeptides. Further, the invention also relates to compositions for laundry, dish wash and/or hard surface cleaning.

### Description of the Related Art

For a number of years α-amylase enzymes have been used for a variety of different purposes, the most important of which are starch liquefaction, textile desizing, starch modification in the paper and pulp industry, and for brewing and baking. A further use of α-amylases, which is becoming increasingly important, is the removal of starchy stains during washing with a detergent at alkaline pH.

Examples of commercial α-amylase products are Termamyl®, Duramyl^{™}, Natalase®, BAN® and Fungamyl®, all available from Novo Nordisk A/S, Denmark. These and similar products from other commercial sources have an acidic to a neutral pH optimum, typically in the range of from pH 5 to pH 7.5, and they do not display optimal activity in detergent solutions at alkaline pH.

WO 95/26397 discloses an α-amylase from a *Bacillus* strain.

WO 96/23873 describes variants of Bacillus amylases with improved performance under washing conditions.

US 5,147,796 describe an alkaline pullulanase having alpha-amylase activity. Fig. 2b of the document shows optimum amylase activity at pH 8-8.5.

M. Takagi et al., J. Ferment. Bioeng., vol 81, No. 6, 557-559 (1996) describe an alkaliphilic alpha-amylase-pullulanase from *Bacillus* sp. The enzyme has optimum amylase activity at pH 9, but the activity drops rapidly at higher pH, and the activity at pH 10 is lower than at pH 7.

WO 97/00324 (KAO) discloses a gene encoding an alkaline liquefying α-amylase derived from *Bacillus* sp. strain KSM-AP1378 with the deposited no. FERM BP-3048 suitable for detergents.

It is an object of the present invention to provide novel α-amylases with improved performance in alkaline solutions, especially in alkaline detergent solutions at pH around 9-11.

### Summary of the Invention

The present invention relates to isolated polypeptides having α-amylase activity and having an amino acid sequence which consists of amino acids 1 to 485 of SEQ ID NO:2 or SEQ ID NO:4.

The present invention also relates to isolated nucleic acid sequences encoding the polypeptides and to nucleic acid constructs, vectors, and host cells comprising the nucleic acid sequences as well as methods for producing and using the polypeptides.

### Brief Description of the Figures

The present invention is further illustrated with reference to the accompanying drawings, in which:
Figure 1 shows the alignment of a number of *Bacillus* α-amylases.
Figure 2 shows the pH Profile of the α-amylases AAI-6 and AAI-10 compared to α-amylases SP722 and SP690. The activity is shown in absolute values as Abs650/mg. The pH profile is measured at 37°C.
Figure 3 shows the Temperature Profile of the α-amylases AAI-6 and AAI-10 at pH 9.0 compared to the reference α-amylases SP722 and SP690.
Figure 4 shows the wash performance of AAI-6 and AAI-10 in the AP Model Detergent 97 in comparison to SP722 , SP690 and Termamyl®.
Figure 5 shows the wash performance of AAI-6 and AAI-10 in Omo Multi Acao in comparison to SP722 , SP690 and Termamyl®.
Figure 6 shows the wash performance of AAI-6 and AAI-10 in Omo Concentrated in comparison to SP722 , SP690 and Termamyl®.
Figure 7 shows the wash performance of AAI-6 and AAI-10 in Ariel Futur liquid in comparison to SP722 , SP690 and Termamyl®.

### Detailed Description of the Invention

### Microbial source

The alkaline α-amylases of the invention may be derived from a strain of *Bacillus.* Preferred strains are of *Bacillus* sp. DSM 12650 (the AAI-6 α-amylase) or DSM 12651 (the AAI-10 α-amylase). These strains were deposited on 25^{th} January 1999 by the inventors under the terms of the Budapest Treaty on the International Recognition of the Deposit of Microorganisms for the Purposes of Patent Procedure at Deutshe Sammmlung von Microorganismen und Zellkulturen GmbH (DSMZ), Mascheroder Weg 1b, D-38124 Braunschweig DE.

*Escherichia coli* strains termed NN049469 and NN049468 containing the α-amylase genes in plasmids pLiH1300 (AAI 6) and pLiH1298 (AAI10), respectively, have also been deposited on 7^{th} April 1999 under the terms of the Budapest Treaty with the Deutshe Sammmlung von Microorganismen und Zellkulturen GmbH (DSMZ), Mascheroder Weg 1b, D-38124 Braunschweig DE, and given the accession numbers DSM12763 and DSM12762, respectively. The above deposited *Bacillus* sp. donor organisms were collected in Iceland in 1997.

### Polypeptides Having α-amylase Activity

α-Amylases (α-1,4-glucan-4-glucanohydrolases, EC 3.2.1.1) constitute a group of enzymes, which catalyze hydrolysis of starch and other linear and branched 1,4-glucosidic oligo- and polysaccharides. For purposes of the present invention, α-amylase activity is determined using the Phadebas assay or the pNPG7 assay described below in the "Materials and Methods" section.

### Homology of Enzyme

The amino acid sequence homology may be determined as the degree of identity between the two sequences indicating a derivation of the first sequence from the second. The homology may suitably be determined by means of computer programs known in the art. Thus, GAP provided in GCG version 8 (Needleman, S.B. and Wunsch, C.D., (1970), Journal of Molecular Biology, 48, 443-453) may be used for a pairwise alignment of the sequences and calculation of the degree of identity or degree of homology using the default settings. Alternatively, Gap from GCG version 9 may be used with a translated version 8 peptide scoring matrix, a gap creation penalty of 30, a gap extension penalty of 1 using ntol's matrix (http://plasmid/∼bioweb/matrix/) without end gap penalty.

### Homology to known Bacillus sp. α-amylases

A homology search of known sequences showed homologies for the sequences of the invention with a number of *Bacillus* amylases in the range 67-81 % on amino acid determined as described above.

Specifically, homologous α-amylases to the sequences of the invention (i.e., SEQ ID NO: 2 and SEQ ID NO: 4) are SP690 (SEQ ID NO: 1 of US patent no. 5,856,164 which is about 81% homologous), SP722 (SEQ ID NO: 2 of US patent no. 5,856,164 which is about 81% homologous) and the mature part (amino acids 31-516) of the α-amylase obtained from *Bacillus* sp. KSM-AP1378 disclosed as SEQ ID NO: 2 of WO 97/00324 which is about 81% homologous to the sequences of the invention.

Preferably, the polypeptides of the present invention comprise the amino acid sequence of SEQ ID NO:2 or SEQ ID NO:4 or an allelic variant thereof; or a fragment thereof that has α-amylase activity. SEQ ID NO: 2 or SEQ ID NO: 4 show the mature part the alkaline α-amylases of the invention.

A fragment of SEQ ID NO:2 or SEQ ID NO: 4 are polypeptides having one or more amino acids deleted from the amino and/or carboxyl terminus of this amino acid sequence.

An allelic variant denotes any of two or more alternative forms of a gene occupying the same chromosomal locus. Allelic variation arises naturally through mutation, and may result in polymorphism within populations. Gene mutations can be silent (no change in the encoded polypeptide) or may encode polypeptides having altered amino acid sequences. An allelic variant of a polypeptide is a polypeptide encoded by an allelic variant of a gene.

The amino acid sequences of the homologous polypeptides may differ from the amino acid sequence of SEQ ID NO:2 or SEQ ID NO:4 by an insertion or deletion of one or more amino acid residues and/or the substitution of one or more amino acid residues by different amino acid residues. Preferably, amino acid changes are of a minor nature, that is conservative amino acid substitutions that do not significantly affect the folding and/or activity of the protein; small deletions, typically of one to about 30 amino acids; small amino- or carboxyl-terminal extensions, such as an amino-terminal methionine residue; a small linker peptide of up to about 20-25 residues; or a small extension that facilitates purification by changing net charge or another function, such as a poly-histidine tract, an antigenic epitope or a binding domain.

Examples of conservative substitutions are within the group of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions, which do not generally alter the specific activity are known in the art and are described, for example, by H. Neurath and R.L. Hill, 1979, In, The Proteins, Academic Press, New York. The most commonly occurring exchanges are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly as well as these in reverse.

In a second embodiment, the present invention relates to isolated polypeptides having α-amylase activity which are encoded by nucleic acid sequences which hybridize under medium stringency conditions, more preferably medium-high stringency conditions, even more preferably high stringency conditions, and most preferably very high stringency conditions with a nucleic acid probe which hybridizes under the same conditions with (i) the nucleic acid sequence of SEQ ID NO:1 or SEQ ID NO:3, (ii) the cDNA sequence of SEQ ID NO:1 or SEQ ID NO:3, (iii) a subsequence of (i) or (ii), or (iv) a complementary strand of (i), (ii), or (iii) (J. Sambrook, E.F. Fritsch, and T. Maniatus, 1989, Molecular Cloning, A Laboratory Manual, 2d edition, Cold Spring Harbor, New York). The subsequence of SEQ ID NO:1 or SEQ ID NO:3 may be at least 100 nucleotides or preferably at least 200 nucleotides. Moreover, the subsequence may encode a polypeptide fragment, which has α-amylase activity. The polypeptides may also be allelic variants or fragments of the polypeptides that have α-amylase activity.

The nucleic acid sequence of SEQ ID NO:1 or SEQ ID NO:3 or a subsequence thereof, as well as the amino acid sequence of SEQ ID NO:2 or SEQ ID NO: 4 or a fragment thereof, may be used to design a nucleic acid probe to identify and clone DNA encoding polypeptides having α-amylase activity from strains of different genera or species according to methods well known in the art. In particular, such probes can be used for hybridization with the genomic or cDNA of the genus or species of interest, following standard Southern blotting procedures, in order to identify and isolate the corresponding gene therein. Such probes can be considerably shorter than the entire sequence, but should be at least 15, preferably at least 25, and more preferably at least 35 nucleotides in length. Longer probes can also be used. Both DNA and RNA probes can be used. The probes are typically labeled for detecting the corresponding gene (for example, with ³²P, ³H, ³⁵S, biotin, or avidin). Such probes are encompassed by the present invention.

Thus, a genomic DNA or cDNA library prepared from such other organisms may be screened for DNA, which hybridizes with the probes described above and which encodes a polypeptide having α-amylase activity. Genomic or other DNA from such other organisms may be separated by agarose or polyacrylamide gel electrophoresis, or other separation techniques. DNA from the libraries or the separated DNA may be transferred to and immobilized on nitrocellulose or other suitable carrier material. In order to identify a clone or DNA which is homologous with SEQ ID NO:1 or SEQ ID NO: 3 or subsequences thereof, the carrier material is used in a Southern blot. For purposes of the present invention, hybridization indicates that the nucleic acid sequence hybridizes to a nucleic acid probe corresponding to the nucleic acid sequence shown in SEQ ID NO:1 or SEQ ID NO: 3, its complementary strand, or a subsequence thereof, under medium to very high stringency conditions. Molecules to which the nucleic acid probe hybridizes under these conditions are detected using X-ray film.

In another preferred embodiment, the nucleic acid probe is the nucleic acid sequence contained in plasmids pLiH1300 (AAI6) or pLiH1298 (AAI10), respectively, which are contained in *Escherichia coli DSM12763or Escherichia coli* DSM12762, respectively, or, wherein the nucleic acid sequence encodes a polypeptide having acid α-amylase activity of the invention and shown in SEQ ID NO: 2 or SEQ ID NO: 4, respectively.

For long probes of at least 100 nucleotides in length, medium to very high stringency conditions are defined as prehybridization and hybridization at 42°C in 5X SSPE, 0.3% SDS, 200 micro g/ml sheared and denatured salmon sperm DNA, 35% formamide for medium and medium-high stringencies, or 50% formamide for high and very high stringencies, following standard Southern blotting procedures.

For long probes of at least 100 nucleotides in length, the carrier material is finally washed three times each for 15 minutes using 2 x SSC, 0.2% SDS preferably at least at 55°C (medium stringency), preferably at least at 60°C (medium-high stringency), more preferably at least at 65°C (high stringency), and most preferably at least at 70°C (very high stringency).

For short probes which are about 15 nucleotides to about 70 nucleotides in length, stringency conditions are defined as prehybridization, hybridization, and washing post-hybridization at 5°C to 10°C below the calculated Tₘ using the calculation according to Bolton and McCarthy (1962, Proceedings of the National Academy of Sciences USA 48:1390) in 0.9 M NaCl, 0.09 M Tris-HCl pH 7.6, 6 mM EDTA, 0.5% NP-40, 1X Denhardt's solution, 1 mM sodium pyrophosphate, 1 mM sodium monobasic phosphate, 0.1 mM ATP, and 0.2 mg of yeast RNA per ml following standard Southern blotting procedures.

For short probes, which are about 15 nucleotides to about 70 nucleotides in length, the carrier material is washed once in 6X SCC plus 0.1% SDS for 15 minutes and twice each for 15 minutes using 6X SSC at 5°C to 10°C below the calculated Tₘ.

In a third embodiment, the present invention relates to isolated polypeptides, i.e., the polypeptides shown in SEQ ID NO: 2 or SEQ ID NO: 4, having the following physicochemical properties:

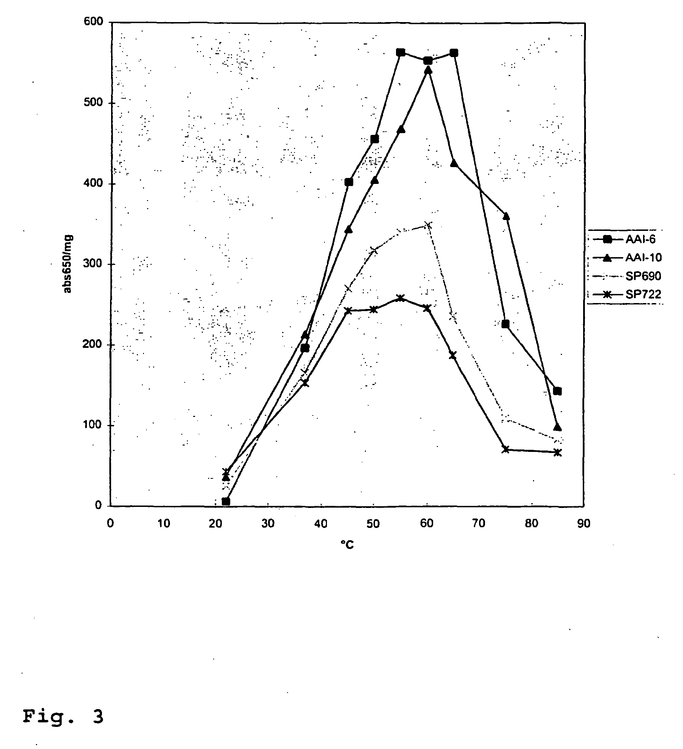

A temperature optimum (see Fig. 3) determined using the Phadebas method (pH 9.0) in the range between 55 and 65°C, more precisely at about 60°C.

A pI for AAI-6 determined using isoelectric focusing was found to be between 7-7.3, and the pI for AAI-10 was found to be between 7-8.

A polypeptide of the present invention may be obtained from microorganisms of any genus. For purposes of the present invention, the term "obtained from" as used herein .in connection with a given source shall mean that the polypeptide encoded by the nucleic acid sequence is produced by the source or by a cell in which the nucleic acid sequence from the source has been inserted.

A polypeptide of the present invention may be a bacterial polypeptide. For example, the polypeptide may be a gram positive bacterial polypeptide such as a *Bacillus* polypeptide, *e.g.,* a *Bacillus alkalophilus*, *Bacillus amyloliquefaciens, Bacillus brevis, Bacillus circulans, Bacillus coagulans, Bacillus lautus*, *Bacillus lentus, Bacillus licheni formis, Bacillus megaterium*, *Bacillus stearothermophilus, Bacillus subtilis,* or *Bacillus thuringiensis* polypeptide; or a *Streptomyces* polypeptide, *e.g.,* a *Streptomyces lividans* or *Streptomyces murinus* polypeptide; or a gram negative bacterial polypeptide, *e.g.,* an *E. coli* or a *Pseudomonas* sp. polypeptide.

In another preferred embodiment, the polypeptide is a *Bacillus* sp. polypeptide, more preferred embodiment, the polypeptide is a *Bacillus* sp. DSM 12650 and *Bacillus* sp. DSM 12651 polypeptide, e.g., the polypeptide with the amino acid sequence of SEQ ID NO: 2 or SEQ ID NO: 4, respectively.

It will be understood that for the aforementioned species, the invention encompasses both the perfect and imperfect states, and other taxonomic equivalents, *e.g*., anamorphs, regardless of the species name by which they are known. Those skilled in the art will readily recognize the identity of appropriate equivalents.

Strains of these species are readily accessible to the public in a number of culture collections, such as the American Type Culture Collection (ATCC), Deutsche Sammlung von Mikroorganismen und Zellkulturen GmbH (DSM), Centraalbureau Voor Schimmelcultures (CBS), and Agricultural Research Service Patent Culture Collection, Northern Regional Research Center (NRRL).

Furthermore, such polypeptides may be identified and obtained from other sources including microorganisms isolated from nature (*e.g*., soil, composts, water, etc.) using the above-mentioned probes. Techniques for isolating microorganisms from natural habitats are well known in the art.

The nucleic acid sequence may then be derived by similarly screening a genomic or cDNA library of another microorganism. Once a nucleic acid sequence encoding a polypeptide has been detected with the probe(s), the sequence may be isolated or cloned by utilizing techniques which are known to those of ordinary skill in the art (see, *e.g*., Sambrook et al., 1989, *supra*).

As defined herein, an "isolated" polypeptide is a polypeptide which is essentially free of other non-α-amylase polypeptides, e.g., at least about 20% pure, preferably at least about 40% pure, more preferably about 60% pure, even more preferably about 80% pure, most preferably about 90% pure, and even most preferably about 95% pure, as determined by SDS-PAGE.

Polypeptides encoded by nucleic acid sequences of the present invention also include fused polypeptides or cleavable fusion polypeptides in which another polypeptide is fused at the N-terminus or the C-terminus of the polypeptide or fragment thereof. A fused polypeptide is produced by fusing a nucleic acid sequence (or a portion thereof) encoding another polypeptide to a nucleic acid sequence (or a portion thereof) of the present invention. Techniques for producing fusion polypeptides are known in the art, and include ligating the coding sequences encoding the polypeptides so that they are in frame and that expression of the fused polypeptide is under control of the same promoter(s) and terminator.

The term "improved wash performance" means in the context of the present invention a performance determined under the washing conditions described in Example 9, which is higher than other alpha-amylase used for washing, e.g., SP690, SP722 and Termamyl®, or in the case of a mutant/variant of the invention in comparison to the parent alpha-amylase, i.e., the unmutated, such as un-substituted alpha-amylase backbone.

### Increased thermostability

In the context of the invention the term "acidic pH" means a pH below 7.0, especially below the pH range, in which industrial starch liquefaction processes are normally performed, which is between pH 5.5 and 6.2.

In the context of the present invention the term "low Calcium concentration" means concentrations below the normal level used in industrial starch liquefaction. Normal concentrations vary depending of the concentration of free Ca²⁺ in the corn. Normally a dosage corresponding to 1 mM (40ppm) is added which together with the level in corn gives between 40 and 60 ppm free Ca²⁺.

In the context of the invention the term "high temperatures" means temperatures between 95°C and 160°C, especially the temperature range in which industrial starch liquefaction processes are normally performed, which is between 95°C and 105°C.

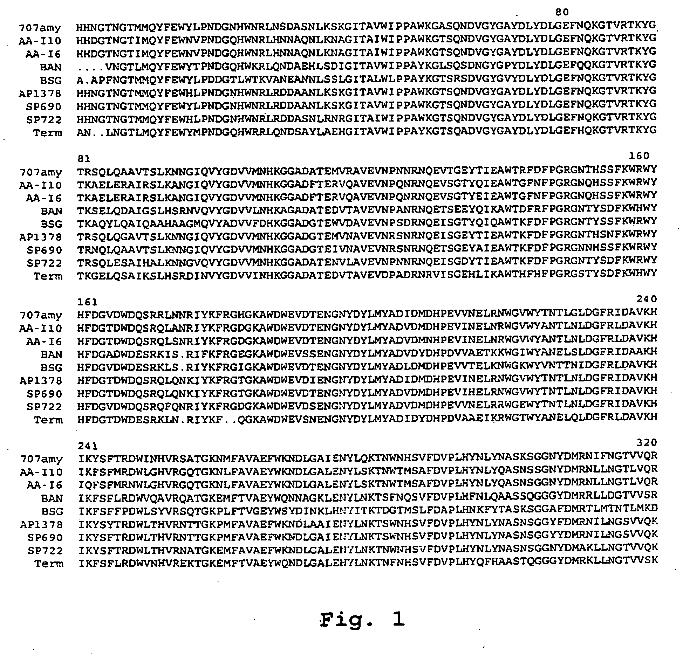

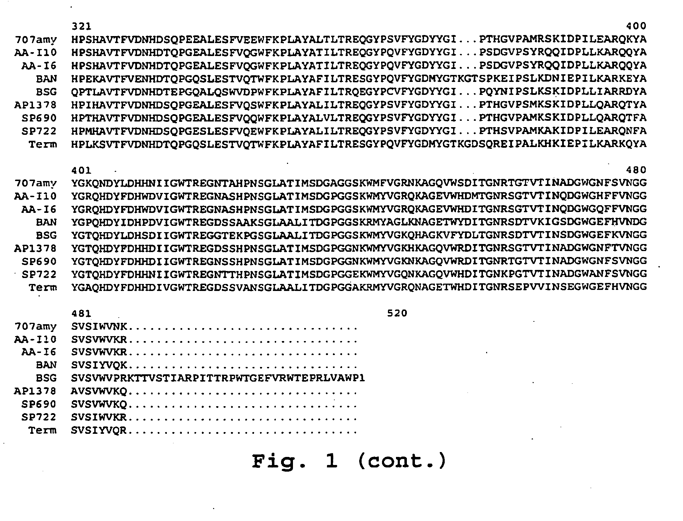

It may be mentioned here that amino acid residues, respectively, at positions corresponding to N195, V206, E212 and E216, respectively, in SEQ ID NO: 2 or SEQ ID NO: 4 constitute amino acid residues, which are conserved in numerous Termamyl-like alpha-amylases, i.e., Termamyl® (*B. licheniformis* alpha-amylase). Thus, for example, the corresponding positions of residues in AAI-10 and AAI-6 and Termamyl can be see in the alignment in Fig. 1 and in Table 1 and Table 2, below.

**Table 1.**

| Termamyl-like alpha-amylase | | | | | |
|---|---|---|---|---|---|
| *B. licheniformis* (Termamyl) | N190 | I201 | H205 | E211 | N265 |
| SP690 | N195 | V206 | H210 | E216 | N270 |
| SP722 | N195 | V206 | H210 | E216 | N270 |
| AAI-10 (SEQ ID NO: 2) | N195 | V206 | H210 | E216 | N270 |
| AAI-6 (SEQ ID NO: 4) | N195 | V206 | H210 | E216 | N270 |

**Table 2.**

| Termamyl-like alpha-amylase | | | | | | |
|---|---|---|---|---|---|---|
| *SP690* | R181, | G182, | T183, | G184, | K185, | A186 |
| *SP722* | R181, | G182, | D183, | G184, | K185, | A186 |
| AAI-10 (SEQ ID NO: 2) | R181, | G182, | D183, | G184, | K185, | A186 |
| AAI-6 (SEQ ID NO: 4) | R181, | G182, | D183, | G184, | K185, | A186 |

### Cloning a DNA sequence encoding an α-amylase

The DNA sequence encoding a parent alpha-amylase as defined above may be isolated from any cell or microorganism producing the alpha-amylase in question, using various methods well known in the art. First, a genomic DNA and/or cDNA library should be constructed using chromosomal DNA or messenger RNA from the organism that produces the alpha-amylase to be studied. Then, if the amino acid sequence of the alpha-amylase is known, homologous, labelled oligonucleotide probes may be synthesized and used to identify alpha-amylase-encoding clones from a genomic library prepared from the organism in question. Alternatively, a labelled oligonucleotide probe containing sequences homologous to a known alpha-amylase gene could be used as a probe to identify alpha-amylase-encoding clones, using hybridization and washing conditions of lower stringency.

Yet another method for identifying alpha-amylase-encoding clones would involve inserting fragments of genomic DNA into an expression vector, such as a plasmid, transforming alpha-amylase-negative bacteria with the resulting genomic DNA library, and then plating the transformed bacteria onto agar containing a substrate for alpha-amylase, thereby allowing clones expressing the alpha-amylase to be identified.

Alternatively, the DNA sequence encoding the enzyme may be prepared synthetically by established standard methods, e.g., the phosphoroamidite method described by S.L. Beaucage and M.H. Caruthers (1981) or the method described' by Matthes et al. (1984). In the phosphoroamidite method, oligonucleotides are synthesized, e.g., in an automatic DNA synthesizer, purified, annealed, ligated and cloned in appropriate vectors.

Finally, the DNA sequence may be of mixed genomic and synthetic origin, mixed synthetic and cDNA origin or mixed genomic and cDNA origin, prepared by ligating fragments of synthetic, genomic or cDNA origin (as appropriate, the fragments corresponding to various parts of the entire DNA sequence), in accordance with standard techniques. The DNA sequence may also be prepared by polymerase chain reaction (PCR) using specific primers, for instance as described in US 4,683,202 or R.K. Saiki et al. (1988).

### Site-directed mutagenesis

Once an alpha-amylase-encoding DNA sequence has been isolated, and desirable sites for mutation identified, mutations may be introduced using synthetic oligonucleotides. These oligonucleotides contain nucleotide sequences flanking the desired mutation sites; mutant nucleotides are inserted during oligonucleotide synthesis. In a specific method, a single-stranded gap of DNA, bridging the alpha-amylase-encoding sequence, is created in a vector carrying the alpha-amylase gene. Then the synthetic nucleotide, bearing the desired mutation, is annealed to a homologous portion of the single-stranded DNA. The remaining gap is then filled in with DNA polymerase I (Klenow fragment) and the construct is ligated using T4 ligase. A specific example of this method is described in Morinaga et al. (1984). US 4,760,025 disclose the introduction of oligonucleotides encoding multiple mutations by performing minor alterations of the cassette. However, an even greater variety of mutations can be introduced at any one time by the Morinaga method, because a multitude of oligonucleotides, of various lengths, can be introduced.

Another method for introducing mutations into alpha-amylase-encoding DNA sequences is described in Nelson and Long (1989). It involves the 3-step generation of a PCR fragment containing the desired mutation introduced by using a chemically synthesized DNA strand as one of the primers in the PCR reactions. From the PCR-generated fragment, a DNA fragment carrying the mutation may be isolated by cleavage with restriction endonucleases and reinserted into an expression plasmid.

### Alternative methods of providing alpha-amylase variants

Alternative methods for providing variants of the invention include gene-shuffling method known in the art including the methods, *e*.*g*., described in WO 95/22625 (from Affymax Technologies N.V.) and WO 96/00343 (from Novo Nordisk A/S).

### Expression of alpha-amylase variants

According to the invention, a DNA sequence encoding the variant produced by methods described above, or by any alternative methods known in the art, can be expressed, in enzyme form, using an expression vector which typically includes control sequences encoding a promoter, operator, ribosome binding site, translation initiation signal, and, optionally, a repressor gene or various activator genes.

The recombinant expression vector carrying the DNA sequence encoding an alpha-amylase variant of the invention may be any vector, which may conveniently be subjected to recombinant DNA procedures, and the choice of vector will often depend on the host cell into which it is to be introduced. Thus, the vector may be an autonomously replicating vector, i.e., a vector which exists as an extrachromosomal entity, the replication of which is independent of chromosomal replication, e.g., a plasmid, a bacteriophage or an extrachromosomal element, minichromosome or an artificial chromosome. Alternatively, the vector may be one which, when introduced into a host cell, is integrated into the host cell genome and replicated together with the chromosome(s) into which it has been integrated.

In the vector, the DNA sequence should be operably connected to a suitable promoter sequence. The promoter may be any DNA sequence, which shows transcriptional activity in the host cell of choice and may be derived from genes encoding proteins either homologous or heterologous to the host cell. Examples of suitable promoters for directing the transcription of the DNA sequence encoding an alpha-amylase variant of the invention, especially in a bacterial host, are the promoter of the *lac* operon of *E*.*coli*, the *Streptomyces coelicolor* agarase gene dagA promoters, the promoters of the *Bacillus licheniformis* alpha-amylase gene (*amyL*), the promoters of the *Bacillus stearothermophilus* maltogenic amylase gene (*amyM*), the promoters of the *Bacillus amyloliquefaciens* alpha-amylase (*amyQ*), the promoters of the Ba*cillus subtilis* xylA and xylB genes etc. For transcription in a fungal host, examples of useful promoters are those derived from the gene encoding *A. oryzae* TAKA amylase, *Rhizomucor miehei* aspartic proteinase, *A. niger* neutral alpha-amylase, *A. niger* acid stable alpha-amylase, *A. niger* glucoamylase, *Rhizomucor miehei* lipase, *A. oryzae* alkaline protease, *A. oryzae* triose phosphate isomerase or *A. nidulans* acetamidase.

The expression vector of the invention may also comprise a suitable transcription terminator and, in eukaryotes, polyadenylation sequences operably connected to the DNA sequence encoding the alpha-amylase variant of the invention. Termination and polyadenylation sequences may suitably be derived from the same sources as the promoter.

The vector may further comprise a DNA sequence enabling the vector to replicate in the host cell in question. Examples of such sequences are the origins of replication of plasmids pUC19, pACYC177, pUB110, pE194, pAMB1 and pIJ702.

The vector may also comprise a selectable marker, e.g. a gene the product of which complements a defect in the host cell, such as the *dal* genes from *B. subtilis* or *B. licheniformis,* or one which confers antibiotic resistance such as ampicillin, kanamycin, chloramphenicol or tetracyclin resistance. Furthermore, the vector may comprise *Aspergillus* selection markers such as amdS, argB, niaD and sC, a marker giving rise to hygromycin resistance, or the selection may be accomplished by co-transformation, e.g., as described in WO 91/17243.

While intracellular expression may be advantageous in some respects, e.g., when using certain bacteria as host cells, it is generally preferred that the expression is extracellular. In general, the *Bacillus* alpha-amylases mentioned herein comprise a preregion permitting secretion of the expressed protease into the culture medium. If desirable, this preregion may be replaced by a different preregion or signal sequence, conveniently accomplished by substitution of the DNA sequences encoding the respective preregions.

The procedures used to ligate the DNA construct of the invention encoding an alpha-amylase variant, the promoter, terminator and other elements, respectively, and to insert them into suitable vectors containing the information necessary for replication, are well known to persons skilled in the art (cf., for instance, Sambrook et al., Molecular Cloning: A Laboratory Manual, 2nd Ed., Cold Spring Harbor, 1989).

The cell of the invention, either comprising a DNA construct or an expression vector of the invention as defined above, is advantageously used as a host cell in the recombinant production of an alpha-amylase variant of the invention. The cell may be transformed with the DNA construct of the invention encoding the variant, conveniently by integrating the DNA construct (in one or more copies) in the host chromosome. This integration is generally considered to be an advantage as the DNA sequence is more likely to be stably maintained in the cell. Integration of the DNA constructs into the host chromosome may be performed according to conventional methods, *e.g.*, by homologous or heterologous recombination. Alternatively, the cell may be transformed with an expression vector as described above in connection with the different types of host cells.

The cell of the invention may be a cell of a higher organism such as a mammal or an insect, but is preferably a microbial cell, *e.g*., a bacterial or a fungal (including yeast) cell.

Examples of suitable bacteria are grampositive bacteria such as *Bacillus subtilis, Bacillus licheniformis, Bacillus lentus, Bacillus brevis, Bacillus stearothermophilus*, *Bacillus alkalophilus, Bacillus amyloliquefaciens, Bacillus coagulans, Bacillus circulans, Bacillus lautus*, *Bacillus megaterium, Bacillus thuringiensis*, or *Streptomyces lividans* or *Streptomyces murinus,* or gramnegative bacteria such as *E*.*coli*. The transformation of the bacteria may, for instance, be effected by protoplast transformation or by using competent cells in a manner known *per se*.

The yeast organism may favourably be selected from a species of *Saccharomyces* or *Schizosaccharomyces, e.g., Saccharomyces cerevisiae*. The filamentous fungus may advantageously belong to a species of *Aspergillus, e.g., Aspergillus oryzae* or *Aspergillus niger.* Fungal cells may be transformed by a process involving protoplast formation and transformation of the protoplasts followed by regeneration of the cell wall in a manner known *per se*. A suitable procedure for transformation of *Aspergillus* host cells is described in EP 238 023.

In yet a further aspect, the present invention relates to a method of producing an alpha-amylase variant of the invention, which method comprises cultivating a host cell as described above under conditions conducive to the production of the variant and recovering the variant from the cells and/or culture medium.

The medium used to cultivate the cells may be any conventional medium suitable for growing the host cell in question and obtaining expression of the alpha-amylase variant of the invention.

Suitable media are available from commercial suppliers or may be prepared according to published recipes (*e.g*., as described in catalogues of the American Type Culture Collection).

The alpha-amylase variant secreted from the host cells may conveniently be recovered from the culture medium by well-known procedures, including separating the cells from the medium by centrifugation or filtration, and precipitating proteinaceous components of the medium by means of a salt such as ammonium sulphate, followed by the use of chromatographic procedures such as ion exchange chromatography, affinity chromatography, or the like.

### Nucleic Acid Sequences

The present invention also relates to isolated nucleic acid sequences, which encode a polypeptide of the present invention. In a preferred embodiment, the nucleic acid sequence is set forth in SEQ ID NO: 1. In another more preferred embodiment, the nucleic acid sequence is the sequence contained in plasmid pLIH1300 (AAI 6) or plasmid LiH1298 (AAI 10), respectively, that is contained in *Escherichia coli* DSM12763 and *Escherichia coli* DSM 12762, respectively. In another preferred embodiment, the nucleic acid sequence is the mature polypeptide coding region of SEQ ID NO: 1 or SEQ ID NO: 3. The present invention also encompasses nucleic acid sequences which encode a polypeptide having the amino acid sequence of SEQ ID NO:2 or SEQ ID NO: 4 which differ from SEQ ID NO: 1 or SEQ ID NO: 3 by virtue of the degeneracy of the genetic code. The present invention also relates to subsequences of SEQ ID NO:1 or SEQ ID NO: 3 which encode fragments of SEQ ID NO: 2 or SEQ ID NO: 4, respectively, that have α-amylase activity.

Subsequences of SEQ ID NO: 1 or SEQ ID NO: 3 are nucleic acid sequences encompassed by SEQ ID NO: 1 or SEQ ID NO: 3 except that one or more nucleotides from the 5' and/or 3' end have been deleted.

The present invention also relates to mutant nucleic acid sequences comprising at least one mutation in the mature polypeptide coding sequence of SEQ ID NO:1 or SEQ ID NO: 3, in which the mutant nucleic acid sequence encodes a polypeptide which consists of amino acids 1 to 485 of SEQ ID NO: 2 or SEQ ID NO: 4.

The techniques used to isolate or clone a nucleic acid sequence encoding a polypeptide are known in the art and include isolation from genomic DNA, preparation from cDNA, or a combination thereof. The cloning of the nucleic acid sequences of the present invention from such genomic DNA can be effected, *e*.*g*., by using the well-known polymerase chain reaction (PCR) or antibody screening of expression libraries to detect cloned DNA fragments with shared structural features. See, e.g., Innis et al., 1990, PCR: A Guide to Methods and Application, Academic Press, New York. Other nucleic acid amplification procedures such as ligase chain reaction (LCR), ligated activated transcription (LAT) and nucleic acid sequence-based amplification (NASBA) may be used. The nucleic acid sequence may be cloned from a strain of *Bacillus,* or another or related organism and thus, for example, may be an allelic or species variant of the polypeptide encoding region of the nucleic acid sequence.

The term "isolated nucleic acid sequence" as used herein refers to a nucleic acid sequence which is essentially free of other nucleic acid sequences, *e.g*., at least about 20% pure, preferably at least about 40% pure, more preferably at least about 60% pure, even more preferably at least about 80% pure, and most preferably at least about 90% pure as determined by agarose electrophoresis. For example, an isolated nucleic acid sequence can be obtained by standard cloning procedures used in genetic engineering to relocate the nucleic acid sequence from its natural location to a different site where it will be reproduced. The cloning procedures may involve excision and isolation of a desired nucleic acid fragment comprising the nucleic acid sequence encoding the polypeptide, insertion of the fragment into a vector molecule, and incorporation of the recombinant vector into a host cell where multiple copies or clones of the nucleic acid sequence will be replicated. The nucleic acid sequence may be of genomic, cDNA, RNA, semisynthetic, synthetic origin, or any combinations thereof.

### Nucleic Acid Constructs

The present invention also relates to nucleic acid constructs comprising a nucleic acid sequence of the present invention operably linked to one or more control sequences, which direct the expression of the coding sequence in a suitable host cell under conditions compatible with the control sequences. Expression will be understood to include any step involved in the production of the polypeptide including, but not limited to, transcription, post-transcriptional modification, translation, post-translational modification, and secretion.

"Nucleic acid construct" is defined herein as a nucleic acid molecule, either single- or double-stranded, which is isolated from a naturally occurring gene or which has been modified to contain segments of nucleic acid which are combined and juxtaposed in a manner which would not otherwise exist in nature. The term nucleic acid construct is synonymous with the term expression cassette when the nucleic acid construct contains all the control sequences required for expression of a coding sequence of the present invention. The term "coding sequence" is defined herein as a portion of a nucleic acid sequence, which directly specifies the amino acid sequence of its protein product. The boundaries of the coding sequence are generally determined by a ribosome binding site (prokaryotes) or by the ATG start codon (eukaryotes) located just upstream of the open reading frame at the 5' end of the mRNA and a transcription terminator sequence located just downstream of the open reading frame at the 3' end of the mRNA. A coding sequence can include, but is not limited to, DNA, cDNA, and recombinant nucleic acid sequences.

An isolated nucleic acid sequence encoding a polypeptide of the present invention may be manipulated in a variety of ways to provide for expression of the polypeptide. Manipulation of the nucleic acid sequence prior to its insertion into a vector may be desirable or necessary depending on the expression vector. The techniques for modifying nucleic acid sequences utilizing recombinant DNA methods are well known in the art.

The term "control sequences" is defined herein to include all components, which are necessary or advantageous for the expression of a polypeptide of the present invention. Each control sequence may be native or foreign to the nucleic acid sequence encoding the polypeptide. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the nucleic acid sequence encoding a polypeptide. The term "operably linked" is defined herein as a configuration in which a control sequence is appropriately placed at a position relative to the coding sequence of the DNA sequence such that the control sequence directs the expression of a polypeptide.

### Promoter Sequence

The control sequence may be an appropriate promoter sequence, a nucleic acid sequence which is recognized by a host cell for expression of the nucleic acid sequence. The promoter sequence contains transcriptional control sequences which mediate the expression of the polypeptide. The promoter may be any nucleic acid sequence which shows transcriptional activity in the host cell of choice including mutant, truncated, and hybrid promoters, and may be obtained from genes encoding extracellular or intracellular polypeptides either homologous or heterologous to the host cell.

Examples of suitable promoters for directing the transcription of the nucleic acid constructs of the present invention, especially in a bacterial host cell, are the promoters obtained from the *E. coli lac* operon, *Streptomyces coelicolor* agarase gene (*dagA*), *Bacillus subtilis* levansucrase gene (*sacB*), *Bacillus licheniformis* alpha-amylase gene (*amyL*), *Bacillus stearothermophilus* maltogenic amylase gene (*amyM*), *Baccillus amyloliquefaciens* alpha-amylase gene (*amyQ*), *Bacillus licheniformis* penicillinase gene (*penP*), *Bacillus subtilis xylA* and *xylB* genes, and prokaryotic beta-lactamase gene (Villa-Kamaroff et al., 1978, Proceedings of the National Academy of Sciences USA 75: 3727-3731), as well as the tac promoter (DeBoer et al., 1983, Proceedings of the National Academy of Sciences USA 80: 21-25). Further promoters are described in "Useful proteins from recombinant bacteria" in Scientific American, 1980, 242: 74-94; and in Sambrook et *al.,* 1989, *supra*.

### Terminator sequence

The control sequence may also be a suitable transcription terminator sequence, a sequence recognized by a host cell to terminate transcription. The terminator sequence is operably linked to the 3' terminus of the nucleic acid sequence encoding the polypeptide. Any terminator, which is functional in the host cell of choice may be used in the present invention.

### Signal Peptide

The control sequence may also be a signal peptide coding region that codes for an amino acid sequence linked to the amino terminus of a polypeptide and directs the encoded polypeptide into the cell's secretory pathway. The 5' end of the coding sequence of the nucleic acid sequence may inherently contain a signal peptide coding region naturally linked in translation reading frame with the segment of the coding region which encodes the secreted polypeptide. Alternatively, the 5' end of the coding sequence may contain a signal peptide coding region which is foreign to the coding sequence. The foreign signal peptide coding region may be required where the coding sequence does not naturally contain a signal peptide coding region. Alternatively, the foreign signal peptide coding region may simply replace the natural signal peptide coding region in order to enhance secretion of the polypeptide. However, any signal peptide coding region which directs the expressed polypeptide into the secretory pathway of a host cell of choice may be used in the present invention.

Effective signal peptide coding regions for bacterial host cells are the signal peptide coding regions obtained from the genes for *Bacillus* NCIB 11837 maltogenic amylase, *Bacillus stearothermophilus* alpha-amylase, *Bacillus licheniformis* subtilisin, *Bacillus licheniformis* beta-lactamase, *Bacillus stearothermophilus* neutral proteases (*nprT*, *nprS*, *nprM*), and *Bacillus subtilis prsA*. Further signal peptides are described by Simonen and Palva, 1993, Microbiological Reviews 57: 109-137.

### Regulatory system

It may also be desirable to add regulatory sequences, which allow the regulation of the expression of the polypeptide relative to the growth of the host cell. Examples of regulatory systems are those which cause the expression of the gene to be turned on or off in response to a chemical or physical stimulus, including the presence of a regulatory compound. Regulatory systems in prokaryotic systems include the *lac, tac,* and *trp* operator systems. In yeast, the ADH2 system or GAL1 system may be used. In filamentous fungi, the TAKA alpha-amylase promoter, *Aspergillus niger* glucoamylase promoter, and *Aspergillus oryzae* glucoamylase promoter may be used as regulatory sequences. Other examples of regulatory sequences are those, which allow for gene amplification. In eukaryotic systems, these include the dihydrofolate reductase gene, which is amplified in the presence of methotrexate, and the metallothionein genes, which are amplified with heavy metals. In these cases, the nucleic acid sequence encoding the polypeptide would be operably linked with the regulatory sequence.

### Expression Vectors

The present invention also relates to recombinant expression vectors comprising a nucleic acid sequence of the present invention, a promoter, and transcriptional and translational stop signals. The various nucleic acid and control sequences described above may be joined together to produce a recombinant expression vector which may include one or more convenient restriction sites to allow for insertion or substitution of the nucleic acid sequence encoding the polypeptide at such sites. Alternatively, the nucleic acid sequence of the present invention may be expressed by inserting the nucleic acid sequence or a nucleic acid construct comprising the sequence into an appropriate vector for expression. In creating the expression vector, the coding sequence is located in the vector so that the coding sequence is operably linked with the appropriate control sequences for expression.

The recombinant expression vector may be any vector (*e.g*., a plasmid or virus) which can be conveniently subjected to recombinant DNA procedures and can bring about the expression of the nucleic acid sequence. The choice of the vector will typically depend on the compatibility of the vector with the host cell into which the vector is to be introduced. The vectors may be linear or closed circular plasmids.

The vector may be an autonomously replicating vector, *i.e*., a vector which exists as an extrachromosomal entity, the replication of which is independent of chromosomal replication, *e.g*., a plasmid, an extrachromosomal element, a minichromosome, or an artificial chromosome. The vector may contain any means for assuring self-replication. Alternatively, the vector may be one which, when introduced into the host cell, is integrated into the genome and replicated together with the chromosome(s) into which it has been integrated. Furthermore, a single vector or plasmid or two or more vectors or plasmids which together contain the total DNA to be introduced into the genome of the host cell, or a transposon may be used.

The vectors of the present invention preferably contain one or more selectable markers which permit easy selection of transformed cells. A selectable marker is a gene the product of which provides for biocide or viral resistance, resistance to heavy metals, prototrophy to auxotrophs, and the like. Examples of bacterial selectable markers are the *dal* genes from *Bacillus subtilis* or *Bacillus licheniformis*, or markers, which confer antibiotic resistance such as ampicillin, kanamycin, chloramphenicol or tetracycline resistance. Suitable markers for yeast host cells are ADE2, HIS3, LEU2, LYS2, MET3, TRP1, and URA3. A selectable marker for use in a filamentous fungal host cell may be selected from the group including, but not limited to, *amdS* (acetamidase), *argB* (ornithine carbamoyltransferase), *bar* (phosphinothricin acetyltransferase), *hygB* (hygromycin phosphotransferase), *niaD* (nitrate reductase), *pyrG* (orotidine-5'-phosphate decarboxylase), *sC* (sulfate adenyltransferase), *trpC* (anthranilate synthase), as well as equivalents thereof. Preferred for use in an *Aspergillus* cell are the *amdS* and *pyrG* genes of *Aspergillus nidulans* or *Aspergillus oryzae* and the *bar* gene of *Streptomyces hygroscopicus.*

The vectors of the present invention preferably contain an element(s) that permits stable integration of the vector into the host cell genome or autonomous replication of the vector in the cell independent of the genome of the cell.

For integration into the host cell genome, the vector may rely on the nucleic acid sequence encoding the polypeptide or any other element of the vector for stable integration of the vector into the genome by homologous or nonhomologous recombination. Alternatively, the vector may contain additional nucleic acid sequences for directing integration by homologous recombination into the genome of the host cell. The additional nucleic acid sequences enable the vector to be integrated into the host cell genome at a precise location(s) in the chromosome(s). To increase the likelihood of integration at a precise location, the integrational elements should preferably contain a sufficient number of nucleic acids, such as 100 to 1,500 base pairs, preferably 400 to 1,500 base pairs, and most preferably 800 to 1,500 base pairs, which are highly homologous with the corresponding target sequence to enhance the probability of homologous recombination. The integrational elements may be any sequence that is homologous with the target sequence in the genome of the host cell. Furthermore, the integrational elements may be non-encoding or encoding nucleic acid sequences. On the other hand, the vector may be integrated into the genome of the host cell by nonhomologous recombination.

For autonomous replication, the vector may further comprise an origin of replication enabling the vector to replicate autonomously in the host cell in question. Examples of bacterial origins of replication are the origins of replication of plasmids pBR322, pUC19, pACYC177, and pACYC184 permitting replication in *E. coli,* and pUB110, pE194, pTA1060, and pAMß1 permitting replication in *Bacillus.* Examples of origins of replication for use in a yeast host cell are the 2micron origin of replication, ARS1, ARS4, the combination of ARS1 and CEN3, and the combination of ARS4 and CEN6. The origin of replication may be one having a mutation which makes its functioning temperature-sensitive in the host cell (see, *e.g.,* Ehrlich, 1978, Proceedings of the National Academy of Sciences USA 75: 1433).

More than one copy of a nucleic acid sequence of the present invention may be inserted into the host cell to increase production of the gene product. An increase, in the copy number of the nucleic acid sequence can be obtained by integrating at least one additional copy of the sequence into the host cell genome or by including an amplifiable selectable marker gene with the nucleic acid sequence where cells containing amplified copies of the selectable marker gene, and thereby additional copies of the nucleic acid sequence, can be selected for by cultivating the cells in the presence of the appropriate selectable agent.

The procedures used to ligate the elements described above to construct the recombinant expression vectors of the present invention are well known to one skilled in the art (see, *e*.*g*., Sambrook *et al*., 1989, *supra*)*.*

### Host Cells

The present invention also relates to recombinant host cells, comprising a nucleic acid sequence of the invention, which are advantageously used in the recombinant production of the polypeptides. A vector comprising a nucleic acid sequence of the present invention is introduced into a host cell so that the vector is maintained as a chromosomal integrant or as a self-replicating extra-chromosomal vector as described earlier.

The term "host cell" encompasses any progeny of a parent cell that is not identical to the parent cell due to mutations that occur during replication. The choice of a host cell will to a large extent depend upon the gene encoding the polypeptide and its source.

The host cell may be a unicellular microorganism, *e.g*., a prokaryote, or a non-unicellular microorganism, *e.g*., a eukaryote.

Useful unicellular cells are bacterial cells such as gram positive bacteria including, but not limited to, a *Bacillus* cell, *e.g., Bacillus alkalophilus*, *Bacillus amyloliquefaciens, Bacillus brevis*, *Bacillus circulans*, *Bacillus clausii, Bacillus coagulans, Bacillus lautus*, *Bacillus lentus*, *Bacillus licheniformis*, *Bacillus megaterium, Bacillus stearothermophilus*, *Bacillus subtilis,* and *Bacillus thuringiensis*; or a *Streptomyces* cell, e.g., *Streptomyces lividans* or *Streptomyces murinus*, or gram negative bacteria such as *E. coli* and *Pseudomonas* sp. In a preferred embodiment, the bacterial host cell is a *Bacillus lentus, Bacillus licheniformis*, *Bacillus stearothermophilus* or *Bacillus subtilis* cell. In another preferred embodiment, the *Bacillus* cell is an alkalophilic *Bacillus*.

The introduction of a vector into a bacterial host cell may, for instance, be effected by protoplast transformation (see, *e*.*g*., Chang and Cohen, 1979, Molecular General Genetics 168: 111-115), using competent cells (see, *e*.*g*., Young and Spizizin, 1961, Journal of Bacteriology 81: 823-829, or Dubnau and Davidoff-Abelson, 1971, Journal of Molecular Biology 56: 209-221), electroporation (see, e.g., Shigekawa and Dower, 1988, Biotechniques 6: 742-751), or conjugation (see, *e.g*., Koehler and Thorne, 1987, Journal of Bacteriology 169: 5771-5278).

### Methods of Production

The present invention also relates to methods for producing a polypeptide of the present invention comprising (a) cultivating a strain, which in its wild-type form is capable of producing the polypeptide, to produce a supernatant comprising the polypeptide; and (b) recovering the polypeptide. Preferably, the strain is of the genus *Bacillus sp.*

The present invention also relates to methods for producing a polypeptide of the present invention comprising (a) cultivating a host cell under conditions conducive for production of the polypeptide; and (b) recovering the polypeptide.

The present invention also relates to methods for producing a polypeptide of the present invention comprising (a) cultivating a host cell under conditions conducive for production of the polypeptide, wherein the host cell comprises a mutant nucleic acid sequence having at least one mutation in the mature polypeptide coding region of SEQ ID NO:1 or SEQ ID NO: 3, wherein the mutant nucleic acid sequence encodes a polypeptide which consists of amino acids 1 to 485 of SEQ ID NO:2 or SEQ ID NO: 4, and (b) recovering the polypeptide.

### Compositions

In a still further aspect, the present invention relates to compositions comprising a polypeptide of the present invention. Preferably, the compositions are enriched in a polypeptide of the present invention. In the present context, the term "enriched" indicates that the α-amylase activity of the composition has been increased, *e.g.,* with an enrichment factor of 1.1.

The composition may comprise a polypeptide of the invention as the major enzymatic component, *e.g*., a mono-component composition. Alternatively, the composition may comprise multiple enzymatic activities, such as an aminopeptidase, amylase, carbohydrase, carboxypeptidase, catalase, cellulase, chitinase, cutinase, cyclodextrin glycosyltransferase, deoxyribonuclease, esterase, alpha-galactosidase, beta-galactosidase, glucoamylase, alpha-glucosidase, beta-glucosidase, haloperoxidase, invertase, laccase, lipase, mannosidase, oxidase, pectinolytic enzyme, peptidoglutaminase, peroxidase, phytase, polyphenoloxidase, proteolytic enzyme, ribonuclease, transglutaminase, or xylanase. The additional enzyme(s) may be producible by means of a microorganism belonging to the genus *Aspergillus*, preferably *Aspergillus aculeatus, Aspergillus awamori, Aspergillus niger,* or *Aspergillus oryzae*, or *Trichoderma, Humicola,* preferably *Humicola insolens,* or *Fusarium,* preferably *Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum*, *Fusarium reticulatum, Fusarium roseum, Fusarium sambucinum, Fusarium sarcochroum, Fusarium sulphureum, Fusarium toruloseum, Fusarium trichothecioides,* or *Fusarium venenatum.*

The polypeptide compositions may be prepared in accordance with methods known in the art and may be in the form of a liquid or a dry composition. For instance, the polypeptide composition may be in the form of a granulate or a microgranulate. The polypeptide to be included in the composition may be stabilized in accordance with methods known in the art.

Examples are given below of preferred uses of the polypeptide compositions of the invention. The dosage of the polypeptide composition of the invention and other conditions under which the composition is used may be determined on the basis of methods known in the art.

### Industrial Applications

Owing to their activity at alkaline pH values, the α-amylases of the invention are well suited for use in a variety of industrial processes, in particular the enzyme finds potential applications as a component in detergents, e.g., laundry, dish wash and hard surface cleaning detergent compositions, but it may also be useful for desizing of textiles, fabrics and garments, beer making or brewing, in pulp and paper production, and further in the production of sweeteners and ethanol, such as fuel, drinking and industrial ethanol, from starch or whole grains.

### Starch Conversion

Conventional starch-conversion processes, such as liquefaction and saccharification processes are described in, e.g., US Patent No. 3,912,590 and EP patent publications Nos. 252,730 and 63,909, hereby incorporated by reference.

### Pulp and Paper Production

The alkaline α-amylases of the invention may also be used in the production of lignocellulosic materials, such as pulp, paper and cardboard, from starch reinforced waste paper and cardboard, especially where repulping occurs at pH above 7 and where amylases can facilitate the disintegration of the waste material through degradation of the reinforcing starch. The α-amylases of the invention is especially useful in a process for producing a papermaking pulp from starch-coated printed paper. The process may be performed as described in WO 95/14807, comprising the following steps:
a) disintegrating the paper to produce a pulp,
b) treating with a starch-degrading enzyme before, during or after step a), and
c) separating ink particles from the pulp after steps a) and b).

The α-amylases of the invention may also be very useful in modifying starch where enzymatically modified starch is used in papermaking together with alkaline fillers such as calcium carbonate, kaolin and clays. With the alkaline a-amylases of the invention it becomes possible to modify the starch in the presence of the filler thus allowing for a simpler integrated process.

### Desizing of Textiles, Fabrics, and Garments

The α-amylases of the invention may also be very useful in textile desizing. In the textile processing industry, a-amylases are traditionally used as auxiliaries in the desizing process to facilitate the removal of starch-containing size, which has served as a protective coating on weft yarns during weaving. Complete removal of the size coating after weaving is important to ensure optimum results in the subsequent processes, in which the fabric is scoured, bleached and dyed. Enzymatic starch breakdown is preferred because it does not involve any harmful effect on the fiber material. In order to reduce processing cost and increase mill throughput, the desizing processing is sometimes combined with the scouring and bleaching steps. In such cases, non-enzymatic auxiliaries such as alkali or oxidation agents are typically used to break down the starch, because traditional alpha-amylases are not very compatible with high pH levels and bleaching agents. The non-enzymatic breakdown of the starch size does lead to some fiber damage because of the rather aggressive chemicals used. Accordingly, it would be desirable to use the alpha-amylases of the invention as they have an improved performance in alkaline solutions. The alpha-amylases may be used alone or in combination with a cellulase when desizing cellulose-containing fabric or textile.

Desizing and bleaching processes are well known in the art. For instance, such processes are described in WO 95/21247, US patent 4,643,736, EP 119,920 hereby in corporate by reference.

Commercially available products for desizing include Aquazyme® and Aquazyme® Ultra from Novo Nordisk A/S.

### Beer making

The α-amylases of the invention may also be very useful in a beer-making process; the α-amylases will typically be added during the mashing process.

### Detergent Compositions

The enzyme of the invention may be added to and thus become a component of a detergent composition.

The detergent composition of the invention may for example be formulated as a hand or machine laundry detergent composition including a laundry additive composition suitable for pre-treatment of stained fabrics and a rinse added fabric softener composition, or be formulated as a detergent composition for use in general household hard surface cleaning operations, or be formulated for hand or machine dishwashing operations.

In a specific aspect, the invention provides a detergent additive comprising the enzyme of the invention. The detergent additive as well as the detergent composition may comprise one or more other enzymes such as a protease, a lipase, a cutinase, an amylase, a carbohydrase, a cellulase, a pectinase, a mannanase, an arabinase, a galactanase, a xylanase, an oxidase, e.g., a laccase, and/or a peroxidase.

In general the properties of the chosen enzyme(s) should be compatible with the selected detergent, (i.e. pH-optimum, compatibility with other enzymatic and non-enzymatic ingredients, etc.), and the enzyme(s) should be present in effective amounts. Proteases: Suitable proteases include those of animal, vegetable or microbial origin. Microbial origin is preferred. Chemically modified or protein engineered mutants are included. The protease may be a serine protease or a metallo protease, preferably an alkaline microbial protease or a trypsin-like protease. Examples of alkaline proteases are subtilisins, especially those derived from *Bacillus,* e.g., subtilisin Novo, subtilisin Carlsberg, subtilisin 309, subtilisin 147 and subtilisin 168 (described in WO 89/06279). Examples of trypsin-like proteases are trypsin (e.g. of porcine or bovine origin) and the *Fusarium* protease described in WO 89/06270 and WO 94/25583.

Examples of useful proteases are the variants described in WO 92/19729, WO 98/20115, WO 98/20116, and WO 98/34946, especially the variants with substitutions in one or more of the following positions: 27, 36, 57, 76, 87, 97, 101, 104, 120, 123, 167, 170, 194, 206, 218, 222, 224, 235 and 274.

Preferred commercially available protease enzymes include Alcalase^{™}, Savinase^{™}, Primase^{™}, Duralase^{™}, Esperase^{™}, and Kannase^{™} (Novo Nordisk A/S), Maxatase^{™}, Maxacal^{™}, Maxapem^{™}, Properase^{™}, Purafect^{™}, Purafect OxP^{™}, FN2^{™}, and FN3^{™} (Genencor International Inc.).

Lipases: Suitable lipases include those of bacterial or fungal origin. Chemically modified or protein engineered mutants are included. Examples of useful lipases include lipases from *Humicola* (synonym *Thermomyces*), e.g. from *H. lanuginosa* (*T. lanuginosus*) as described in EP 258 068 and EP 305 216 or from *H. insolens* as described in WO 96/13580, a *Pseudomonas* lipase, e.g., from *P. alcaligenes* or *P. pseudoalcaligenes* (EP 218 272), *P. cepacia* (EP 331 376), *P. stutzeri* (GB 1,372,034), P. *fluorescens, Pseudomonas sp.* strain SD 705 (WO 95/06720 and WO 96/27002), *P. wisconsinensis* (WO 96/12012), a *Bacillus* lipase, e.g. from *B. subtilis* (Dartois et al. (1993), Biochemica et Biophysica Acta, 1131, 253-360), *B. stearothermophilus* (JP 64/744992) or *B*. *pumilus* (WO 91/16422).

Other examples are lipase variants such as those described in WO 92/05249, WO 94/01541, EP 407 225, EP 260 105, WO 95/35381, WO 96/00292, WO 95/30744, WO 94/25578, WO 95/14783, WO 95/22615, WO 97/04079 and WO 97/07202.

Preferred commercially available lipase enzymes include Lipolase^{™} and Lipolase Ultra^{™} (Novo Nordisk A/S).
Amylases: Suitable amylases (α and/or β) include those of bacterial or fungal origin. Chemically modified or protein engineered mutants are included. Amylases include, for example, α-amylases obtained from *Bacillus,* e.g. a special strain of *B*. *licheniformis,* described in more detail in GB 1,296,839.

Examples of useful amylases are the variants described in WO 94/02597, WO 94/18314, WO 96/23873, and WO 97/43424, especially the variants with substitutions in one or more of the following positions: 15, 23, 105, 106, 124, 128, 133, 154, 156, 181, 188, 190, 197, 202, 208, 209, 243, 264, 304, 305, 391, 408, and 444.

Commercially available amylases are Duramyl^{™}, Termamyl^{™}, Fungamyl^{™} and BAN^{™} (Novo Nordisk A/S), Rapidase^{™} and Purastar^{™} (from Genencor International Inc.).
Cellulases: Suitable cellulases include those of bacterial or fungal origin. Chemically modified or protein engineered mutants are included. Suitable cellulases include cellulases from the genera *Bacillus, Pseudomonas*, *Humicola, Fusarium, Thielavia, Acremonium,* e.g. the fungal cellulases produced from *Humicola insolens*, *Myceliophthora thermophila* and *Fusarium oxysporum* disclosed in US 4,435,307, US 5,648,263, US 5,691,178, US 5,776,757 and WO 89/09259.

Especially suitable cellulases are the alkaline or neutral cellulases having colour care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and PCT/DK98/00299.

Commercially available cellulases include Celluzyme^{™}, and Carezyme^{™} (Novo Nordisk A/S), Clazinase^{™}, and Puradax HA^{™} (Genencor International Inc.), and KAC-500(B)^{™} (Kao Corporation).
Peroxidases/Oxidases: Suitable peroxidases/oxidases include those of plant, bacterial or fungal origin. Chemically modified or protein engineered mutants are included. Examples of useful peroxidases include peroxidases from *Coprinus,* e.g. from C. *cinereus*, and variants thereof as those described in WO 93/24618, WO 95/10602, and WO 98/15257.

Commercially available peroxidases include Guardzyme^{™} (Novo Nordisk A/S).

The detergent enzyme(s) may be included in a detergent composition by adding separate additives containing one or more enzymes, or by adding a combined additive comprising all of these enzymes. A detergent additive of the invention, i.e. a separate additive or a combined additive, can be formulated e.g. as a granulate, a liquid, a slurry, etc. Preferred detergent additive formulations are granulates, in particular non-dusting granulates, liquids, in particular stabilized liquids, or slurries.

Non-dusting granulates may be produced, e.g., as disclosed in US 4,106,991 and 4,661,452 and may optionally be coated by methods known in the art. Examples of waxy coating materials are poly(ethylene oxide) products (polyethyleneglycol, PEG) with mean molar weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591. Liquid enzyme preparations may, for instance, be stabilized by adding a polyol such as propylene glycol, a sugar or sugar alcohol, lactic acid or boric acid according to established methods. Protected enzymes may be prepared according to the method disclosed in EP 238,216.

The detergent composition of the invention may be in any convenient form, e.g., a bar, a tablet, a powder, a granule, a paste or a liquid. A liquid detergent may be aqueous, typically containing up to 70 % water and 0-30 % organic solvent, or non-aqueous.

The detergent composition comprises one or more surfactants, which may be non-ionic including semi-polar and/or anionic and/or cationic and/or zwitterionic. The surfactants are typically present at a level of from 0.1% to 60% by weight.

When included therein the detergent will usually contain from about 1% to about 40% of an anionic surfactant such as linear alkylbenzenesulfonate, alpha-olefinsulfonate, alkyl sulfate (fatty alcohol sulfate), alcohol ethoxysulfate, secondary alkanesulfonate, alpha-sulfo fatty acid methyl ester, alkyl- or alkenylsuccinic acid or soap.

When included therein the detergent will usually contain from about 0.2% to about 40% of a non-ionic surfactant such as alcohol ethoxylate, nonylphenol ethoxylate, alkylpolyglycoside, alkyldimethylamineoxide, ethoxylated fatty acid monoethanolamide, fatty acid monoethanolamide, polyhydroxy alkyl fatty acid amide, or N-acyl N-alkyl derivatives of glucosamine ("glucamides").

The detergent may contain 0-65 % of a detergent builder or complexing agent such as zeolite, diphosphate, triphosphate, phosphonate, carbonate, citrate, nitrilotriacetic acid, ethylenediaminetetraacetic acid, diethylenetriaminepentaacetic acid, alkyl- or alkenylsuccinic acid, soluble silicates or layered silicates (e.g. SKS-6 from Hoechst).

The detergent may comprise one or more polymers. Examples are carboxymethylcellulose, poly(vinylpyrrolidone), poly (ethylene glycol), poly(vinyl alcohol), poly(vinylpyridine-N-oxide), poly(vinylimidazole), polycarboxylates such as polyacrylates, maleic/acrylic acid copolymers and lauryl methacrylate/acrylic acid copolymers.

The detergent may contain a bleaching system which may comprise a H₂O₂ source such as perborate or percarbonate which may be combined with a peracid-forming bleach activator such as tetraacetylethylenediamine or nonanoyloxybenzenesulfonate. Alternatively, the bleaching system may comprise peroxyacids of e.g. the amide, imide, or sulfone type.

The enzyme(s) of the detergent composition of the invention may be stabilized using conventional stabilizing agents, e.g., a polyol such as propylene glycol or glycerol, a sugar or sugar alcohol, lactic acid, boric acid, or a boric acid derivative, e.g., an aromatic borate ester, or a phenyl boronic acid derivative such as 4-formylphenyl boronic acid, and the composition may be formulated as described in, e.g., WO 92/19709 and WO 92/19708.

The detergent may also contain other conventional detergent ingredients such as, e.g., fabric conditioners including clays, foam boosters, suds suppressors, anti-corrosion agents, soil-suspending agents, anti-soil redeposition agents, dyes, bactericides, optical brighteners, hydrotropes, tarnish inhibitors, or perfumes.

It is at present contemplated that in the detergent compositions any enzyme, in particular the enzyme of the invention, may be added in an amount corresponding to 0.01-100 mg of enzyme protein per liter of wash liqour, preferably 0.05-5 mg of enzyme protein per liter of wash liqour, in particular 0.1-1 mg of enzyme protein per liter of wash liqour.

The enzyme of the invention may additionally be incorporated in the detergent formulations disclosed in WO 97/07202, which is hereby incorporated as reference.

### Dishwash Deterget Compositions

The enzyme of the invention mat also be used in dish wash detergent compositions, including the following:

| 1) POWDER AUTOMATIC DISHWASHING COMPOSITION | |
|---|---|
| Nonionic surfactant | 0.4 - 2.5% |
| Sodium metasilicate | 0 - 20% |
| Sodium disilicate | 3 - 20% |
| Sodium triphosphate | 20 - 40% |
| Sodium carbonate | 0 - 20% |
| Sodium perborate | 2 - 9% |
| Tetraacetyl ethylene diamine (TAED) | 1 - 4% |
| Sodium sulphate | 5 - 33% |
| Enzymes | 0.0001 - 0.1% |

| 2) POWDER AUTOMATIC DISHWASHING COMPOSITION | |
|---|---|
| Nonionic surfactant (e.g. alcohol ethoxylate) | 1 - 2% |
| Sodium disilicate | 2 - 30% |
| Sodium carbonate | 10 - 50% |
| Sodium phosphonate | 0 - 5% |
| Trisodium citrate dihydrate | 9 - 30% |
| Nitrilotrisodium acetate (NTA) | 0 - 20% |
| Sodium perborate monohydrate | 5 - 10% |
| Tetraacetyl ethylene diamine (TAED) | 1 - 2% |
| Polyacrylate polymer (e.g. maleic acid/acrylic acid copolymer) | 6 - 25% |
| Enzymes | 0.0001 - 0.1% |
| Perfume | 0.1 - 0.5% |
| Water | 5 - 10 |

| 3) POWDER AUTOMATIC DISHWASHING COMPOSITION | |
|---|---|
| Nonionic surfactant | 0.5 - 2.0% |
| Sodium disilicate | 25 - 40% |
| Sodium citrate | 30 - 55% |
| Sodium carbonate | 0 - 29% |
| Sodium bicarbonate | 0 - 20% |
| Sodium perborate'monohydrate | 0 - 15% |
| Tetraacetyl ethylene diamine (TAED) | 0 - 6% |
| Maleic acid/acrylic Acid copolymer | 0 - 5% |
| Clay | 1 - 3% |
| Polyamino acids | 0 - 20% |
| Sodium polyacrylate | 0 - 8% |
| Enzymes | 0.0001 - 0.1% |

| 4) POWDER AUTOMATIC DISHWASHING COMPOSITION | |
|---|---|
| Nonionic surfactant | 1 - 2% |
| Zeolite MAP | 15 - 42% |
| Sodium disilicate | 30 - 34% |
| Sodium citrate | 0 - 12% |
| Sodium carbonate | 0 - 20% |
| Sodium perborate monohydrate | 7 - 15% |
| Tetraacetyl ethylene diamine (TAED) | 0 - 3% |
| Polymer | 0 - 4% |
| Maleic acid/acrylic acid copolymer | 0 - 5% |
| Organic phosphonate | 0 - 4% |
| Clay | 1 - 2% |
| Enzymes | 0.0001 - 0.1% |
| Sodium sulphate | Balance |

| 5) POWDER AUTOMATIC DISHWASHING COMPOSITION | |
|---|---|
| Nonionic surfactant | 1 - 7% |
| Sodium disilicate | 18 - 30% |
| Trisodium citrate | 10 - 24% |
| Sodium carbonate | 12 - 20% |
| Monopersulphate (2 KHSO₅.KHSO₄.K₂SO₄) | 15 - 21% |
| Bleach stabilizer | 0.1 - 2% |
| Maleic acid/acrylic acid copolymer | 0 - 6% |
| Diethylene triamine pentaacetate, pentasodium salt | 0 - 2.5% |
| Enzymes | 0.0001 - 0.1% |
| Sodium sulphate, water | Balance |

| 6) POWDER AND LIQUID DISHWASHING COMPOSITION WITH CLEANING SURFACTANT SYSTEM | |
|---|---|
| Nonionic surfactant | 0 - 1.5% |
| Octadecyl dimethylamine N-oxide dihydrate | 0 - 5% |
| 80:20 wt.C18/C16 blend of octadecyl dimethylamine N-oxide dihydrate and hexadecyldimethyl amine N-oxide dihydrate | 0 - 4% |
| 70:30 wt.C18/C16 blend of octadecyl bis (hydroxyethyl)amine N-oxide anhydrous and hexadecyl bis hydroxyethyl)amine N-oxide anhydrous | 0 - 5% |
| C₁₃-C₁₅ alkyl ethoxysulfate with an average degree of ethoxylation of 3 | 0 - 10% |
| C₁₂-C₁₅ alkyl ethoxysulfate with an average degree of ethoxylation of 3 | 0 - 5% |
| C₁₃-C₁₅ ethoxylated lcohol with an average degree of ethoxylation of 12 | 0 - 5% |
| A blend of C₁₂-C₁₅ ethoxylated alcohols with an average degree of ethoxylation of 9 | 0 - 6.5% |
| A blend of C₁₃-C₁₅ ethoxylated alcohols with an average degree of ethoxylation of 30 | 0 - 4% |
| Sodium disilicate | 0 - 33% |
| Sodium tripolyphosphate | 0 - 46% |
| Sodium citrate | 0 - 28% |
| Citric acid | 0 - 29% |
| Sodium carbonate | 0 - 20% |
| Sodium perborate monohydrate | 0 - 11.5% |
| Tetraacetyl ethylene diamine (TAED) | 0 - 4% |
| Maleic acid/acrylic acid copolymer | 0 - 7.5% |
| Sodium sulphate | 0 - 12.5% |
| Enzymes | 0.0001 - 0.1% |

| 7) NON-AQUEOUS LIQUID AUTOMATIC DISHWASHING COMPOSITION | |
|---|---|
| Liquid nonionic surfactant (e.g. alcohol ethoxylates) | 2.0 - 10.0% |
| Alkali metal silicate | 3.0 - 15.0% |
| Alkali metal phosphate | 20.0 - 40.0% |
| Liquid carrier selected from higher glycols, polyglycols, polyoxides, glycolethers | 25.0 - 45.0% |
| Stabilizer (e.g. a partial ester of phosphoric acid and a C₁₆-C₁₈ alkanol) | 0.5 - 7.0% |
| Foam suppressor (e.g. silicone) | 0 - 1.5% |
| Enzymes | 0.0001 - 0.1% |

| 8) NON-AQUEOUS LIQUID DISHWASHING COMPOSITION | |
|---|---|
| Liquid nonionic surfactant (e.g. alcohol ethoxylates) | 2.0 - 10.0% |
| Sodium silicate | 3.0 - 15.0% |
| Alkali metal carbonate | 7.0 - 20.0% |
| Sodium citrate | 0.0 - 1.5% |
| Stabilizing system (e.g. mixtures of finely divided silicone and low molecular weight dialkyl polyglycol ethers) | 0.5 - 7.0% |
| Low molecule weight polyacrylate | |
| polymer | 5.0 - 15.0% |
| Clay gel thickener (e.g. bentonite) | 0.0 - 10.0% |
| Hydroxypropyl cellulose polymer | 0.0 - 0.6% |
| Enzymes | 0.0001 - 0.1% |
| Liquid carrier selected from higher | |
| lycols, polyglycols, polyoxides and glycol ethers | Balance |

| 9) THIXOTROPIC LIQUID AUTOMATIC DISHWASHING COMPOSITION | |
|---|---|
| C₁₂-C₁₄ fatty acid | 0 - 0.5% |
| Block co-polymer surfactant | 1.5 - 15.0% |
| Sodium citrate | 0 - 12% |
| Sodium tripolyphosphate | 0 - 15% |
| Sodium carbonate | 0 - 8% |
| Aluminium tristearate | 0 - 0.1% |
| Sodium cumene sulphonate | 0 - 1.7% |
| Polyacrylate thickener | 1.32 - 2.5% |
| Sodium polyacrylate | 2.4 - 6.0% |
| Boric acid | 0 - 4.0% |
| Sodium formate | 0 - 0.45% |
| Calcium formate | 0 - 0.2% |
| Sodium n-decydiphenyl oxide disulphonate | 0 - 4.0% |
| Monoethanol amine (MEA) | 0 - 1.86% |
| Sodium hydroxide (50%) | 1.9 - 9.3% |
| 1,2-Propanediol | 0 - 9.4% |
| Enzymes | 0.0001 - 0.1% |
| Suds suppressor, dye, perfumes, water | Balance |

| 10) LIQUID AUTOMATIC DISHWASHING COMPOSITION | |
|---|---|
| Alcohol ethoxylate | 0 - 20% |
| Fatty acid ester sulphonate | 0 - 30% |
| Sodium dodecyl sulphate | 0 - 20% |
| Alkyl polyglycoside | 0 - 21% |
| Oleic acid | 0 - 10% |
| Sodium disilicate monohydrate | 18 - 33% |
| Sodium citrate dihydrate | 18 - 33% |
| Sodium stearate | 0 - 2.5% |
| Sodium perborate monohydrate | 0 - 13% |
| Tetraacetyl ethylene diamine (TAED) | 0 - 8% |
| Maleic acid/acrylic acid copolymer | 4 - 8% |
| Enzymes | 0.0001 - 0.1% |

| 11) LIQUID AUTOMATIC DISHWASHING COMPOSITION CONTAINING PROTECTED BLEACH PARTICLES | |
|---|---|
| Sodium silicate | 5 - 10% |
| Tetrapotassium pyrophosphate | 15 - 25% |
| Sodium triphosphate | 0 - 2% |
| Potassium carbonate | 4 - 8% |
| Protected bleach particles, e.g. chlorine | 5 - 10% |
| Polymeric thickener | 0.7 - 1.5% |
| Potassium hydroxide | 0 - 2% |
| Enzymes | 0.0001 - 0.1% |
| Water | Balance |

11) Automatic dishwashing compositions as described in 1), 2), 3), 4), 6) and 10), wherein perborate is replaced by percarbonate.
12) Automatic dishwashing compositions as described in 1) - 6) which additionally contain a manganese catalyst. The manganese catalyst may, e.g., be one of the compounds described in "Efficient manganese catalysts for low-temperature bleaching", Nature 369, 1994, pp. 637-639.

### Uses

The present invention is also directed to methods for using the polypeptides having α-amylase activity in detergent compositions; in particular laundry detergent compositions and dish wash detergent compositions, hard surface cleaning compositions, and in composition for desizing of textiles, fabrics, or garments, for production of pulp and paper, beer making, and starch conversion processes as described above.

The present invention is further described by the following examples, which should not be construed as limiting the scope of the invention.

### Materials and Methods

Chemicals used as buffers and substrates were commercial products of at least reagent grade.

### Materials:

### Enzymes:

SP690: α-amylase disclosed in SEQ ID NO: 1 of US patent no. 5,856,164.
SP722: α-amylase disclosed in SEQ ID NO: 2 of US patent no. US patent no. 5,856,164.
Termamyl®: α-amylase from *Bacillus licheniformis* disclosed in
SEQ ID NO: 1 of US patent no. 5,830,837.
AAI-10: α-amylase of the invention shown in SEQ ID NO: 2.
AAI-6: α-amylase of the invention shown in SEQ ID NO: 4.

### Model detergent:

A/P (Asia/Pacific) Model Detergent has the following composition: 20% STPP (sodium tripolyphosphate), 25% Na₂SO₄, 15% Na₂CO₃, 20% LAS (linear alkylbenzene sulfonate, Nansa 80S), 5% C₁₂-C₁₅ alcohol ethoxylate (Dobanol 25-7), 5% Na₂Si₂O₅, 0.3% NaCl.
Omo Multi Acao (Brazil),
Omo concentrated powder (EU) (Unilever)
Ariel Futur liquid (EU) (Procter and Gamble)

### Deposit of Biological Material

The following biological material has been deposited under the terms of the Budapest Treaty with the Deutshe Sammmlung von Microorganismen und Zellkulturen GmbH (DSMZ), Mascheroder Weg 1b, D-38124 Braunschweig DE, and given the following accession number:

| Deposit | Accession Number | Date of Deposit |
|---|---|---|
| NN17562 | DSM 12650 | 25^{th} January 1999; |
| NN17563 | DSM 12651 | 25^{th} January 1999; |
| NN049469 | DSM12763 | 7^{th} April 1999; |
| NN049468 | DSM12762 | 7^{th} April 1999; |

The strains have been deposited under conditions that assure that access to the culture will be available during the pendency of this patent application to one determined by the Commissioner of Patents and Trademarks to be entitled thereto under 37 C.F.R. §1.14 and 35 U.S.C. §122. The deposit represents a substantially pure culture of the deposited strain. The deposit is available as required by foreign patent laws in countries wherein counterparts of the subject application, or its progeny are filed. However, it should be understood that the availability of a deposit does not constitute a license to practice the subject invention in derogation of patent rights granted by governmental action.

### Host organism:

Strain SHa273 disclosed in WO 95/10603
*E. coli* strain SJ2 (Diderichsen et al. (1990)), J. Bacteriol., vol. 172, pp. 4315-4321.

### Plasmids:

The gene bank vector was pSJ1678 which is further disclosed in WO 94/19454 which is hereby incorporated by reference.

### Methods

### General molecular biology methods:

Unless otherwise mentioned the DNA manipulations and transformations were performed using standard methods of molecular biology (Sambrook et al. (1989); Ausubel et al. (1995); Harwood and Cutting (1990).

### Fermentation of alpha-amylases and variants

Fermentation may be performed by methods well known in the art or as follows.

A *B*. *subtilis* strain harboring the relevant expression plasmid is streaked on a LB-agar plate with 10 micro g/ml Kanamycin from -80°C stock, and grown overnight at 37°C. The colonies are transferred to 100 ml BPX media supplemented with 10 micro g/ml kanamycin in a 500 ml shaking flask. Composition of BPX medium:

| | |
|---|---|
| Potato starch | 100 g/l |
| Barley flour | 50 g/l |
| BAN 5000 SKB | 0.1 g/l |
| Sodium caseinate | 10 g/l |
| Soy Bean Meal | 20 g/l |
| Na₂HPO₄, 12 H₂O | 9 g/l |
| Pluronic^{™} | 0.1 g/l |

The culture is shaken at 37°C at 270 rpm for 5 days.

Cells and cell debris are removed from the fermentation broth by centrifugation at 4500 rpm in 20-25 minutes. Afterwards the supernatant is filtered to obtain a completely clear solution. The filtrate is concentrated and washed on a UF-filter (10000 cut off membrane) and the buffer is changed to 20mM Acetate pH 5.5. The UF-filtrate is applied on a S-sepharose F.F. and elution is carried out by step elution with 0.2M NaCl in the same buffer.

The eluate is dialysed against 10mM Tris, pH 9.0 and applied on a Q-sepharose F.F. and eluted with a linear gradient from 0-0.3M NaCl over 6 column volumes. The fractions, which contain the activity (measured by the Phadebas assay) are pooled, pH was adjusted to pH 7.5 and remaining color was removed by a treatment with 0.5% W/vol. active coal in 5 minutes.

### Assays for α-Amylase Activity

### 1. Phadebas assay

α-amylase activity is determined by a method employing Phadebas® tablets as substrate. Phadebas tablets (Phadebas® Amylase Test, supplied by Pharmacia Diagnostic) contain a crosslinked insoluble blue-colored starch polymer, which has been mixed with bovine serum albumin and a buffer substance and tabletted.

For every single measurement one tablet is suspended in a tube containing 5 ml 50 mM Britton-Robinson buffer (50 mM acetic acid, 50 mM phosphoric acid, 50 mM boric acid, 0.1 mM CaCl₂, pH adjusted to the' value of interest with NaOH). The test is performed in a water bath at the temperature of interest. The α-amylase to be tested is diluted in x ml of 50 mM Britton-Robinson buffer. 1 ml of this α-amylase solution is added to the 5 ml 50 mM Britton-Robinson buffer. The starch is hydrolyzed by the α-amylase giving soluble blue fragments. The absorbance of the resulting blue solution, measured spectrophotometrically at 620 nm, is a function of the α-amylase activity.

It is important that the measured 620 nm absorbance after 10 or 15 minutes of incubation (testing time) is in the range of 0.2 to 2.0 absorbance units at 620 nm. In this absorbance range there is linearity between activity and absorbance (Lambert-Beer law). The dilution of the enzyme must therefore be adjusted to fit this criterion. Under a specified set of conditions (temp., pH, reaction time, buffer conditions) 1 mg of a given α-amylase will hydrolyze a certain amount of substrate and a blue colour will be produced. The colour intensity is measured at 620 nm. The measured absorbance is directly proportional to the specific activity (activity/mg of pure α-amylase protein) of the α-amylase in question under the given set of conditions.

### 2. Alternative method

α-amylase activity is determined by a method employing the PNP-G7 substrate. PNP-G7 which is a abbreviation for p-nitrophenyl-α,D-maltoheptaoside is a blocked oligosaccharide which can be cleaved by an endo-amylase. Following the cleavage, the α-Glucosidase included in the kit digest the substrate to liberate a free PNP molecule which has a yellow colour and thus can be measured by visible spectophometry at λ=405nm. (400-420 nm.). Kits containing PNP-G7 substrate and α-Glucosidase is manufactured by Boehringer-Mannheim (cat.No. 1054635).

To prepare the substrate one bottle of substrate (BM 1442309) is added to 5 ml buffer (BM1442309). To prepare the α-Glucosidase one bottle of α-Glucosidase (BM 1462309) is added to 45 ml buffer (BM1442309). The working solution is made by mixing 5 ml α-Glucosidase solution with 0.5 ml substrate.

The assay is performed by transforming 20µl enzyme solution to a 96 well microtitre plate and incubating at 25°C. 200 µl working solution, 25°C is added. The solution is mixed and preincubated 1 minute and absorption is measured every 15 sec. over 3 minutes at OD 405 nm.

From the slope of the time dependent absorption-curve, the specific activity (activity per mg enzyme) of the α-amylase in question under the given set of conditions.

### Measurement of the calcium- and pH-dependent stability

Normally industrial liquefaction processes runs using pH 6.0-6.2 as liquefaction pH and an addition of 40 ppm free calcium in order to improve the stability at 95°C-105°C. Some of the herein proposed substitutions have been made in order to improve the stability at
1. lower pH than pH 6.2 and/or
2. at free calcium levels lower than 40 ppm free calcium.

Two different methods can be used to measure the alterations in stability obtained by the different substitutions in the alpha-amylases in question:
Method 1. One assay which measures the stability at reduced pH, pH 5.0, in the presence of 5 ppm free calcium. 10 micro g of the variant are incubated under the following conditions: A 0,1 M acetate solution, pH adjusted to pH 5.0, containing 5ppm calcium and 5% w/w common corn starch (free of calcium). Incubation is made in a water bath at 95°C for 30 minutes.
Method 2. One assay, which measure the stability in the absence of free calcium and where the pH is maintained at pH 6.0. This assay measures the decrease in calcium sensitivity: 10 micro g of the variant were incubated under the following conditions: A 0.1 M acetate solution, pH adjusted to pH 6.0, containing 5% w/w common corn starch (free of calcium). Incubation was made in a water bath at 95°C for 30 minutes.

### Stability determination

All the stability trials are made using the same set up. The method is:
The enzyme is incubated under the relevant conditions (1-4). Samples are taken at 0, 5, 10, 15 and 30 minutes and diluted 25 times (same dilution for all taken samples) in assay buffer (0.1M 50mM Britton buffer pH 7.3) and the activity is measured using the Phadebas assay (Pharmacia) under standard conditions pH 7.3, 37°C.
The activity measured before incubation (0 minutes) is used as reference (100%). The decline in percent is calculated as a function of the incubation time.

### Specific activity determination.

The specific activity is determined using the Phadebas assay (Pharmacia) as activity/mg enzyme.

### Jet cooking and liquefaction with alpha-amylase and variants

Liquefaction experiments are run in the mini-jet system using a dosage of 50 NU/g DS at pH 5.5 with 5 ppm added Ca⁺⁺, to compare the performance of formulated alpha-amylase variant with that of parent alpha-amylase. The reaction is monitored by measuring the DE increase (Neocuproine method) as a function of time.

Cornstarch slurries are prepared by suspending about 11.8 kg Cerestar C*Pharm GL 03406 (89 % starch) in deionized water and making up to 30 kg. The pH is adjusted to 5.5 at ambient temperature, after the addition of 0.55 g CaCl₂. 2H₂O.

An amount of enzyme corresponding to 50 NU/g DS is added, and the conductivity adjusted to 300mS using NaCl. Standard conditions are as follows:

| | |
|---|---|
| Substrate concentration | 35 % w/w (initial) |
| | 31.6-31.9 % w/w (final) |
| Temperature | 105°C, 5 minutes (Primary liquefaction) |
| | 95°C, 90 minutes (Secondary liquefaction) |
| pH (initial) | 5.5 |

After jetting, the liquefied starch is collected and transported in sealed thermos-flasks from the pilot plant to the laboratory, where secondary liquefaction is continued at 95°C.

10 ml samples are taken at 15 minutes intervals from 15-90 minutes. 2 drops of 1 N HCl are added to inactivate the enzyme. From these samples, 0.3-0.1 g (according to the expected DE) are weighed out and diluted to 100 ml. Reducing sugars are then determined according to the Neocuproine method (Determination of reducing sugar with improved precision. Dygert, Li, Florida and Thomas (1965). Anal. Biochem 13, 368) and DE values determined. The development of DE as a function of time is compared.

### Desizing materials:

| | |
|---|---|
| Standard fabrics: | TS526, twill weave, 100% cotton. |
| Impregnation: | 55°C, 60 ppm CaCl₂, pH 6.5 |
| Enzyme solutions: | parent AAI-10 or AAI-6 (1 g/l) |
| Dosages: | 0.05, 0.1, 0.2, 0.5 and 2.0 g/l of the enzyme solutions in the impregnation liquor. |
| Incubation: | 2 hours at 55 or 65°C |
| | 22 hours at 30°C |
| Wash: | 10 minutes in water |
| Drying: | Room temperature |
| Procedures: | Evaluation of Desizing Results - Violet Scale (TEGEWA). |

### Filter screening assays

The assay can be used to screening of alpha-amylase variants having an improved stability at high pH compared to the parent enzyme, and alpha-amylase variants having an improved stability at high pH and medium temperatures compared to the parent enzyme depending of the screening temperature setting

### High pH filter assay

*Bacillus* libraries are plated on a sandwich of cellulose acetate (OE 67, Schleicher & Schuell, Dassel, Germany) - and nitrocellulose filters (Protran-Ba 85, Schleicher & Schuell, Dassel, Germany) on TY agar plates with 10 micro g/ml kanamycin at 37°C for at least 21 hours. The cellulose acetate layer is located on the TY agar plate.

Each filter sandwich is specifically marked with a needle after plating, but before incubation in order to be able to localize positive variants on the filter and the nitrocellulose filter with bound variants is transferred to a container with glycin-NaOH buffer, pH 8.6-10.6 and incubated at room temperature (can be altered from 10°-60°C) for 15 min. The cellulose acetate filters with colonies are stored on the TY-plates at room temperature until use. After incubation, residual activity is detected on plates containing 1% agarose, 0.2% starch in glycin-NaOH buffer, pH 8.6-10.6. The assay plates with nitrocellulose filters are marked the same way as the filter sandwich and incubated for 2 hours. at room temperature. After removal of the filters the assay plates are stained with 10% Lugol solution. Starch degrading variants are detected as white spots on dark blue background and then identified, on the storage plates. Positive variants are rescreened twice under the same conditions as the first screen.

### Low calcium filter assay

The *Bacillus* library are plated on a sandwich of cellulose acetate (OE 67, Schleicher & Schuell, Dassel, Germany) - and nitrocellulose filters (Protran-Ba 85, Schleicher & Schuell, Dassel, Germany) on TY agar plates with a relevant antibiotic, e.g., kanamycin or chloramphenicol, at 37°C for at least 21 hours. The cellulose acetate layer is located on the TY agar plate.

Each filter sandwich is specifically marked with a needle after plating, but before incubation in order to be able to localize positive variants on the filter and the nitrocellulose filter with bound variants is transferred to a container with carbonate/bicarbonate buffer pH 8.5-10 and with different EDTA concentrations (0.001 mM - 100 mM). The filters are incubated at room temperature for 1 hour. The cellulose acetate filters with colonies are stored on the TY-plates at room temperature until use. After incubation, residual activity is detected on plates containing 1% agarose, 0.2% starch in carbonate/bicarbonate buffer pH 8.5-10. The assay plates with nitrocellulose filters are marked the same way as the filter sandwich and incubated for 2 hours. at room temperature. After removal of the filters the assay plates are stained with 10% Lugol solution. Starch degrading variants are detected as white spots on dark blue background and then identified on the storage plates. Positive variants are re-screened twice under the same conditions as the first screen.

### EXAMPLES

### Example 1

### Isolation of genomic DNA of Strain DSM 12560 and DSM 12651

The strains *Bacillus sp*. DSM 12650 (the AAI-6 α-amylase) and *Bacillus sp*. DSM 12651(the AAI-10 α-amylase) were propagated in liquid TY medium (as described in Ausubel et al.(1995)). After 16 hours incubation at 37°C and 300 rpm, the cells were harvested, and genomic DNA isolated by the method described by Pitcher et al. (1989).

### Genomic library construction

Genomic DNA of strains above were partially digested with restriction enzyme Sau3A, and.size-fractionated by electrophoresis on a 0.7 % agarose gel. Fragments between 2 and 10 kb in size was isolated by electrophoresis onto DEAE-cellulose paper (Dretzen et al. (1981).

Isolated DNA fragments were ligated to BamHI digested pSJ1678 plasmid DNA, and the ligation mixture was used to transform E. *coli* SJ2.

### Transformation

*E*. *coli* SJ2 host cells were prepared for and transformed by electroporation using a gene PULSER^{™} electroporator from BIO-RAD as described by the supplier.

### Identification of positive transformants:

DNA libraries in *E*. *coli* SJ2, constructed as described above, were screened on LB agar plates (described in Ausbel et al.(1995)) containing 0.5 % AZCL-amylose (Megazyme) and 10 micro g/ml Chloramphenicol and incubated overnight at 37°C. Clones expressing amylase activity appeared with blue diffusion haloes. One such clone expressing AAI-6 amylase was named LiH1300. Another clone expressing AAI-10 amylase was named LiH1298. The DNA was further characterized by DNA sequencing of part of the cloned Sau3A DNA fragments.

### Example 2

### Determination of the DNA sequence of the gene encoding alpha amylase from strain DSM 12650 (AAI-6).

The clone constituting a large chromosomal fragment containing the gene encoding the amylolytic activity inserted into plasmid pSJ1678, pLiH1300, was used as template in an attempt to specifically PCR amplify internal DNA fragments of the amylase encoding gene by the use of degenerate primers directed towards the conserved regions in known *Bacillus* α-amylases.

The degenerate primers were directed towards the following regions/amino acid sequences:
For36: GITA(L/V/I)W(I/L) (SEQ ID NO: 5)
For97: VY(G/A)D(V/F/L)V(M/L/I/F)NH (SEQ ID NO: 6)
For227: DG(F/I)R(F/L/I/V)DA(A/V)KH (SEQ ID NO: 7)
Rev235: DG(F/I)R(F/L/I/V)DA(A/V)KH (SEQ ID NO: 8)
Rev328: VTFV(D/E)NHD (SEQ ID NO: 9)
Rev410: GWTREG (SEQ ID NO: 10)

The various combinations of forward (For) and reverse (Rev) primers were used in PCR and internal DNA fragments could be amplified.
The DNA fragments were purified by QIAquick spin colums (QUIGEN) and sequenced utilizing the same degenerate primers.
From this initial sequence the DNA sequence of the complete coding region of AAI-6 α-amylase SEQ ID NO:2 was determined by a standard primers-walking approach.

### Example 3

### Determination of the DNA sequence of the gene encoding alpha-amylase from strain DSM 12651 (AAI-10):

The clone constituting a large chromosomal fragment containing the gene encoding the amylolytic activity inserted into plasmid pSJ1678, pLiH1298, was used as template in an attempt to specifically PCR amplify internal DNA fragments of the amylase encoding gene by the use of degenerate primers directed towards the conserved regions in known *Bacillus* alpha-amylases.

The degenerate primers were directed towards the following regions/amino acid sequences:
For36: GITA(L/V/I)W(I/L) (SEQ ID NO: 5)
For97: VY(G/A)D(V/F/L)V(M/L/I/F)NH (SEQ ID NO: 6)
For227: DG(F/I)R(F/L/I/V)DA(A/V)KH (SEQ ID NO: 7)
Rev235: DG(F/I)R(F/L/I/V)DA(A/V)KH (SEQ ID NO: 8)
Rev328: VTFV(D/E)NHD (SEQ ID NO: 9)
Rev410: GWTREG (SEQ ID NO: 10)

The various combinations of forward (For) and reverse (Rev) primers were used in PCR and internal DNA fragments could be amplified.

The DNA fragments were purified by QIAquick spin colums (QUIGEN) and sequenced utilizing the same degenerate primers.

From this initial sequence the DNA sequence (SEQ ID NO: 1) of the complete coding region of AAI-10 α-amylase (SEQ ID NO: 4) was determined by a standard primers-walking approach.

### Example 4

### Subcloning of DSM 12650(AAI-6) α-amylase into pTVB110.

pTVB110 is a plasmid replicating in Bacillus subtilis by the use of origin of replication from pUB110 (Gryczan, T.J. (1978) J. Bact 134:318-329). The plasmid further encodes the cat gene, conferring resistance towards chlorampenicol, obtained from plasmid pC194 (Horinouchi, S. and Weisblum, B. (1982), J. Bact. 150: 815-825). The plasmid harbors a truncated version of the *Bacillus licheniformis* alpha amylase gene, *amyL,* such that the amyL promoter, signal sequence and transcription terminator are present but the plasmid does not provide an amy-plus phenotype (halo formation on starch containing agar).

In order to express high amount of the AAI-6 amylase the mature gene was fused precisely to the *amyL* signal sequence such that transcriptions is initiated by the *amyL* promoter and translocation is directed by the *amyL* signal sequence.

Primers 197cloningN and 197cloningC were used to amplify an approximately 1480 bp fragment by PCR on plasmid pLiH1300 using the Pwo polymerase under conditions recommended by the manufacturer (Boehringer Mannheim).

The resulting approximately 1480 bp fragment was digested with restriction endonucleases PstI and SfiI and ligated with plasmid digested with the same enzymes.

Protease and amylase deleted *Bacillus subtilis* strain SHa273 (disclosed in WO95/10603) was transformed with the ligation mixture and the DNA sequence of an amy-plus transformant was verified. This plasmid is denoted pTVB230.

### Oligonucleotides:

197cloningC:
   5' CCG AGC TCG GCC GGC TGG GCC GTC GAC TTA TCG TTT CAC CCA TAC GG (SEQ ID NO: 11)
197cloningN:
   5' CAT TCT GCA GCA GCG GCG CAT CAC GAT GGG ACG AAC GG (SEQ ID NO: 12)

### Example 5

### Subcloning of AAI-10 alpha amylase into pTVB110.

DNA sequencing revealed a rather high identity between alpha-amylases from stains AAI-6 and AAI-10. Consequently the same oligonucleotides and strategy was utilized for the cloning of AAI-10 alpha-amylase into expression vector pTVB110 resulting in plasmid pTVB229, which was then fermented using standard techniques.

### Example 6

### Purification of the AAI-6 α-amylase

The culture broth was flocculated by adding 0.01 ml 50%(w/w) CaCl₂, 2H₂0, 0.ml 12% (w/w) Sodium aluminate, 0.025 ml 10% C521 and 0.075 ml 0.1% A130 pr. ml culture broth. A clear solution was obtained after centrifugation. The enzyme solution was added ammonium sulphate to a final concentration of 1.2 M and applied on a Butyl Toyo Pearl column (100 ml) previously equilibrated in 1.2 M ammonium sulphate, 10 mM Tris-HCl, pH 7.0. The amylase was eluted using 5 mM Tris-HCl, pH 7.0 and the eluted pool was dialysed against 5 mM Tris-HCl over night. The fraction was then subjected to ion exchange chromatography using a Q-Sepharose column (200 ml) previously equilibrated in 20 mM Tris-HCl, pH 9.0. Unbound material was washed out with the equilibration buffer, and the amylase was eluted using a linear gradient 0 - 1 M NaCl, 20 mM Tris-HCl, pH 9.0. Purity of the amylase preparation was above 95% judged by SDS-PAGE.

### Example 7

### Purification of AAI-10 α-amylase

The culture broth was flocculated as described above. The enzyme solution was added ammonium sulphate to a final concentration of 1.2 M and applied on a Butyl Toyo Pearl column (100 ml) previously equilibrated in 1.2 M ammonium sulphate, 10 mM Tris-HCl, pH 7.0. 20% of the activity was eluted with 5 mM Tris-HCl, pH 7.0 and the rest with isopropanol. The combined pool was fractionated on a Q-Sepharose column (100 ml), pH 9.7. Unbound material was washed out with the equilibration buffer, and the amylase was eluted using a linear gradient 0 - 1 M NaCl, 20 mM Tris-HCl, pH 9.4. The amylase containing pool was finally fractionated on a S Sepharose column, pH 5.5. Purity of the amylase preparation was above 95% judged by SDS-PAGE.

### Example 8

### Characterization of the AAI-6 and AAI-10 α-amylases:

The α-amylase activity was measured using both the Phadebas assay (37°C, pH 7.3) and the Alternative pNPG7 Assay (25°C, pH 7.1) described above. pH- and temperature profiles were made at selected pH- and temperature values. The pH-profile was measured at 37°C and the temperature profile was measured at pH 9.0

Isoelectric Point was determined using isoelectric focusing (Pharmacia, Ampholine, pH 3.5-9.3).

**Table 1. Specific activity and pI.**

| Enzyme | mg/ml | NU/mg Phadebas 37°C, pH7.3 | NU (T) /mg pNPG7, 25°C, pH7.1 | PI |
|---|---|---|---|---|
| AAI-10 (SEQ NO:4) | 0.4 | 17,500 | 12,000 | 7-8 |
| AAI-6 (SEQ ID NO:2) | 0.6 | 25,000 | 13,500 | 7-7.3 |
| SP690 (SEQ ID NO: 1 of US Patent No. 5,856,164) | 8.2 | 35,000 | 6,000 | 7-9 |
| SP722 (SEQ ID NO: 2 of US Patent No. 5,856,164) | 2. | 35,000 | 7,000 | 5-6 |
| Termamyl® | 12.4 | 7,000 | 7,000 | |

| | | | | |
|---|---|---|---|---|
| E = 3.0 cm⁻¹ *(g/l)⁻¹ for AAI-6 and AAI-10 | | | | |

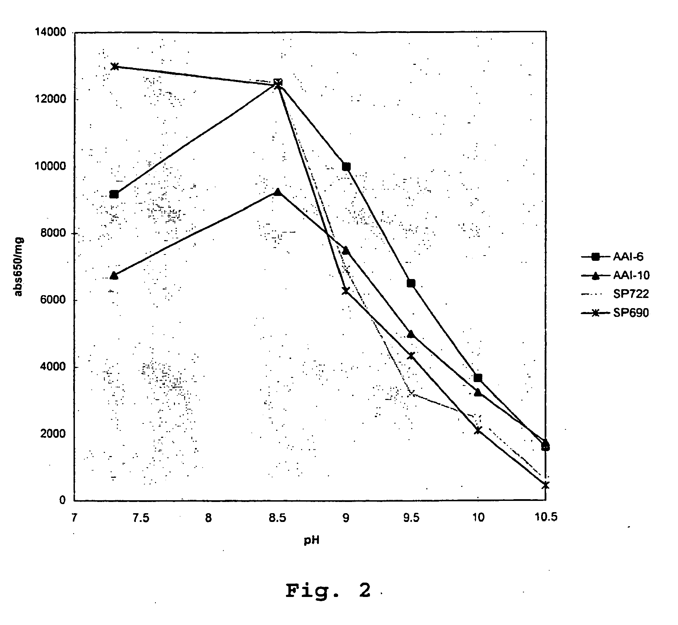

The result of the pH-optimum determination and temperature optimum determination is shown in Figure 2 and Figure 3, respectively.

### Example 9

### Washing test

Washing performance was evaluated by washing soiled test swatches at 15 and 30 minutes at 25 and 40°C respectively in detergent solutions with the AAI-6 and AAI-10 α-amylase of the invention.

The detergents used are disclosed in Table 3 below. The A/P Model Detergent is described' in the Materials section above. The other detergents are commercially available detergents. Commercial detergents containing amylase were inactivated by microwaves before wash.

The purified recombinant AAI-6 and AAI-10 α-amylases of Example 6 and 7 were added to the detergent solutions at the concentration indicated below. The test swatches were soiled with orange rice starch (CS-28 swatches available from CFT, Center for Test Material, Holland).

After washing, the swatches were evaluated by measuring the remission at 460 nm using an Elrepho Remission Spectrophotometer. The results are expressed as ΔR = remission of the swatch washed with the alpha-amylase minus the remission of a swatch washed at the same conditions without the alpha-amylase.

**Table 3. Detergents and wash conditions.**

| Area | Detergent | Det. Dose g/l | Inacti Vation | Enzyme dose mg/l | Temp. °C | Time min | pH | Water hardnes s °dH | Ca: Mg |
|---|---|---|---|---|---|---|---|---|---|
| A/P | Model detergent 97 | 3 | - | 1 | 25 | 15 | 10.5 | 6 | 2:1 |
| Latin America | Omo Multi Acao | 3 | - | 1 | 25 | 15 | 10.6 | 6 | 2:1 |
| Europe | Omo conc. powder | 4 | + | 0.2 | 40 | 30 | 10.2 | 15 | 4:1 |
| Europe | Ariel Futur liquid | 5 | + | 0.2 | 40 | 30 | 9.0 | 15 | 4:1 |

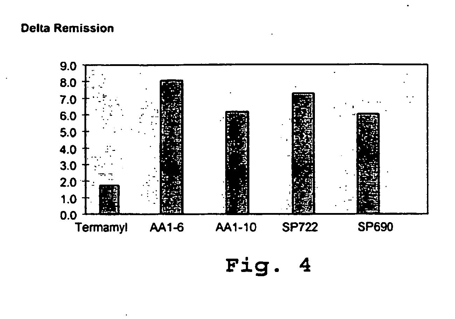

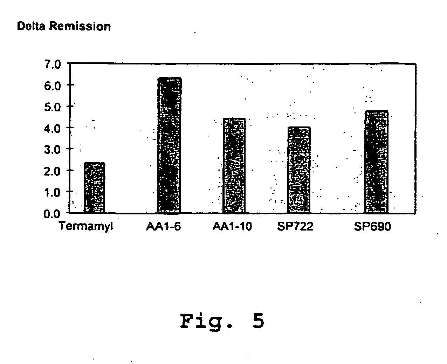

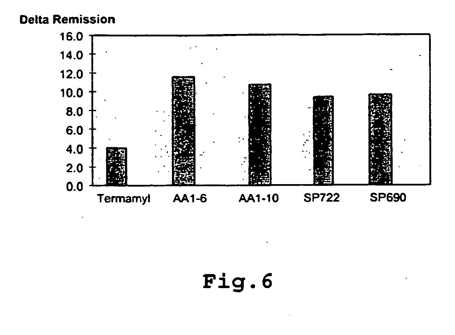

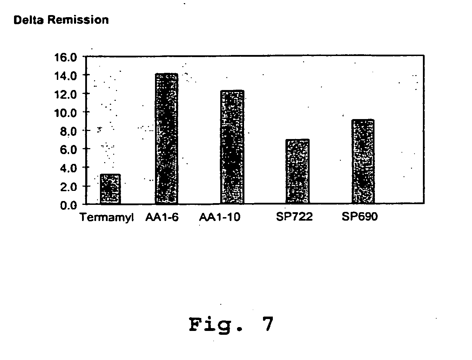

The results are shown in Figures 4-7. The results demonstrate that the α-amylase of the invention is effective in both detergents at highly alkaline pH.

### Example 10

### Desizing test with the AAI-10

The parent AAI-10 alpha-amylase are used for desizing of fabrics using the TEGEWA method (Method and standard scales obtainable from Verband TEGEWA, Karlstrasse 21, Frankfurt a.M., Germany). The conditions are described in the "Materials & Methods" section.

The AAI-10 alpha-amylase is used in desizing tests carried out at 30 and 55°C. The enzyme is dosed from 0.05 to 2 g/l in the impregnation solution. The after-washing procedure is carried out by washing the fabrics in water - instead of the usually hot soda wash.

The effect of is measured using the Violet Scale (TEGEWA), TEGEWA scale: 1-9.

### References

Ausubel, F. M. et al. (eds.); Current protocols in Molecular Biology; 1995; John Wiley and Sons.
Sambrook et al.; Molecular Cloning: A Laboratory Manual; 1989,; Cold Spring Harbor Lab.; Cold Spring Harbor; NY.
Harwood C. R., and Cutting S. M. (eds.); Molecular Biological Methods for Bacillus; 1990; John Wiley and Sons.
Diderichsen B., Wedsted U., Hedegaard L., Jensen B. R., Sjøholm C.; Cloning of aldB, which encodes alpha-acetolactate decarboxylase, an exoenzyme from Bacillus brevis; J. Bacteriol., 1990, vol. 172, pp. 4315-4321.
Pitcher D. G., Saunders N. A., Owen R. J.; Rapid extraction of bacterial genomic DNA with guanidium thiocyanate; Lett. Appl. Microbiol.; 1989; vol. 8; pp. 151-156.
Dretzen G., Bellard M., Sassone-Corsi P., Chambon P.; A reliable method for the recovery of DNA fragments from agarose and acrylamide gels; Anal. Biochem.; 1981; vol. 112; pp. 295-298.

## Claims

1. An isolated polypeptide having α-amylase activity and having an amino acid sequence which consists of amino acids 1 to 485 of SEQ ID NO:2 or SEQ ID NO:4.

2. The polypeptide of claim 1, which is encoded by the nucleic acid sequence contained in plasmid ppLiH1300 or pLiH1298 contained in *E*. *coli* DSM12769 or *E*. *coli* DSM 12768, respectively.

3. An isolated nucleic acid sequence comprising a nucleic acid sequence which encodes the polypeptide of any of claims 1-2.

4. An isolated nucleic acid sequence comprising a nucleic acid sequence having at least one mutation in the mature polypeptide coding sequence of SEQ ID NO:1 or SEQ ID NO: 3, in which the mutant nucleic acid sequence encodes a polypeptide consisting of amino acids 1 to 485 of SEQ ID NO: 2 or SEQ ID NO: 4.

5. The isolated nucleic acid sequence of claim 4, produced by (a) hybridizing a DNA under medium stringency conditions with (i) the nucleic acid sequence of SEQ ID NO:1, (ii) the cDNA sequence of SEQ ID NO:1, (iii) a subsequence of (i) or (ii) of at least 100 nucleotides, or (iv) a complementary strand of (i), (ii), or (iii); and (b) isolating the nucleic acid sequence.

6. A recombinant expression vector comprising the nucleic acid sequence of any of claims 3-5.

7. A recombinant host cell comprising the nucleic acid construct of claim 6.

8. A method for producing a mutant nucleic acid sequence, comprising (a) introducing at least one mutation into the mature polypeptide coding sequence of SEQ ID NO:1 or SEQ ID NO: 3, wherein the mutant nucleic acid sequence encodes a polypeptide consisting of amino acids 1 to 485 of SEQ ID NO:2 or SEQ ID NO: 4; and (b) recovering the mutant nucleic acid sequence.

9. A mutant nucleic acid sequence produced by the method of claim 8.

10. A method for producing a polypeptide, comprising (a) cultivating a strain comprising the mutant nucleic acid sequence of claim 9 encoding the polypeptide to produce a supernatant comprising the polypeptide; and (b) recovering the polypeptide.

11. A method for producing the polypeptide of any of claim 1-2 comprising (a) cultivating a strain to produce a supernatant comprising the polypeptide; and (b) recovering the polypeptide.

12. A composition, in particular a detergent composition, comprising a polypeptide of any of claims 1-2.

13. Use of the polypeptide of any of claims 1-2 in a detergent composition, in particular a laundry detergent composition and a dish wash detergent composition and hard surface cleaning compositions, or in a desizing composition.

## Patentansprüche

1. Isoliertes Polypeptid mit α-Amylaseaktivität und einer Aminosäuresequenz, die aus den Aminosäuren 1 bis 485 der SEQ ID NR: 2 oder SEQ ID NR: 4 besteht.

2. Polypeptid nach Anspruch 1, welches durch die in Plasmid ppLiH1300 oder pLiH1298 enthaltende Nukleinsäuresequenz, enthaltend in *E. coli* DSM12769 bzw. *E. coli* DSM 12768, kodiert ist.

3. Isolierte Nukleinsäuresequenz, umfassend eine Nukleinsäuresequenz, die das Polypeptid nach einem beliebigen der Ansprüche 1-2 kodiert.

4. Isolierte Nukleinsäuresequenz, umfassend eine Nukleinsäuresequenz mit mindestens einer Mutation in der das reife Polypeptid kodierenden Sequenz von SEQ ID NR: 1 oder SEQ ID NR: 3, in der die mutierte Nukleinsäuresequenz ein Polypeptid bestehend aus den Aminosäuren 1 bis 485 der SEQ ID NR: 2 oder SEQ ID NR: 4 kodiert.

5. Die isolierte Nukleinsäuresequenz nach Anspruch 4, erzeugt durch (a) Hybridisieren einer DNA unter Bedingungen mittlerer Stringenz mit (i) der Nukleinsäuresequenz von SEQ ID NR: 1, (ii) der cDNA Sequenz von SEQ ID NR: 1, (iii) einer Teilsequenz von (i) oder (ii) von mindestens 100 Nukleotiden, oder (iv) einem komplementären Strang von (i), (ii), oder (iii); und (b) Isolieren der Nukleinsäuresequenz.

6. Rekombinanter Expressionsvektor umfassend die Nukleinsäuresequenz nach einem beliebigen der Ansprüche 3-5.

7. Rekombinante Wirtszelle umfassend das Nukleinsäurekonstrukt nach Anspruch 6.

8. Verfahren zur Erzeugung einer mutierten Nukleinsäuresequenz, umfassend (a) das Einführen von mindestens einer Mutation in die das reife Polypeptid kodierende Sequenz von SEQ ID NR: 1 oder SEQ ID NR: 3, wobei die mutierte Nukleinsäuresequenz ein Polypeptid bestehend aus den Aminosäuren 1 bis 485 der SEQ ID NR: 2 oder SEQ ID NR: 4 kodiert; und (b) Gewinnen der mutierten Nukleinsäuresequenz.

9. Mutierte Nukleinsäuresequenz, erzeugt durch das Verfahren nach Anspruch 8.

10. Verfahren zur Erzeugung eines Polypeptids, umfassend (a) Kultivieren eines Stammes umfassend die mutierte Nukleinsäuresequenz nach Anspruch 9, die das Polypeptid zur Erzeugung eines Überstandes, umfassend das Polypeptid, kodiert; und (b) Gewinnen des Polypeptids.

11. Verfahren zur Erzeugung eines Polypeptids nach einem beliebigen der Ansprüche 1-2, umfassend (a) Kultivieren eines Stammes, um einen Überstand, umfassend das Polypeptid, zu erzeugen; und (b) Gewinnen des Polypeptids.

12. Zusammensetzung, insbesondere eine Detergenzzusammensetzung, umfassend ein Polypeptid nach einem beliebigen der Ansprüche 1-2.

13. Verwendung des Polypeptids nach einem beliebigen der Ansprüche 1-2 in einer Detergenszusammensetzung, insbesondere einer Waschmittelzusammensetzung und einer Geschirrspülmittelzusammensetzung und einer für harte Oberflächen geeigneten Reinigungsmittelzusammensetzung oder in einer Entschlichtungszusammensetzung.

## Revendications

1. Polypeptide isolé ayant une activité α-amylase et ayant une séquence d'acides aminés qui consiste en les acides aminés 1 à 485 de la SEQ ID NO : 2 ou de la SEQ ID NO : 4.

2. Polypeptide selon la revendication 1, qui est codé par la séquence d'acides nucléiques contenue dans le plasmide ppLiH1300 ou pLiH1298 contenu dans E. Coli DSM12769 ou E. Coli DSM 12768, respectivement.

3. Séquence d'acides nucléiques isolée comprenant une séquence d'acides nucléiques codant le polypeptide de l'une quelconque des revendications 1 et 2.

4. Séquence d'acides nucléiques isolée comprenant une séquence d'acides nucléiques ayant au moins une mutation dans la séquence codant le polypeptide mature de la SEQ ID NO : 1 ou la SEQ ID NO : 3, dans laquelle la séquence d'acides nucléiques mutante code un polypeptide consistant en les acides aminés 1 à 485 de la SEQ ID NO : 2 ou de la SEQ ID NO : 4.

5. Séquence d'acides nucléiques isolée selon la revendication 4, produite par (a) hybridation de l'ADN dans des conditions de stringence moyenne avec (i) la séquence d'acides nucléiques de la SEQ ID NO : 1, (ii) la séquence d'ADNc de la SEQ ID NO : 1, (iii) une sous-séquence de (i) ou (ii) d'au moins 100 nucléotides, ou (iv) un brin complémentaire de (i), (ii), ou (iii) ; et (b) isolation de la séquence d'acides nucléiques.

6. Vecteur d'expression recombinant comprenant la séquence en acides nucléiques selon l'une quelconque des revendications 3 à 5.

7. Cellule hôte recombinante comprenant le construit d'acides nucléiques de la revendication 6.

8. Procédé de production d'une séquence d'acides nucléiques mutante comprenant (a) l'introduction d'au moins une mutation dans la séquence codant le polypeptide mature de la SEQ ID NO : 1 ou de la SEQ ID NO : 3, dans laquelle la séquence d'acides nucléiques mutante code pour un polypeptide consistant en les acides aminés 1 à 485 de la SEQ ID NO : 2 ou de la SEQ ID NO : 4 ; et (b) la récupération de la séquence d'acides nucléiques mutante.

9. Séquence d'acides nucléiques mutante produite par le procédé selon la revendication 8.

10. Procédé de production de polypeptide, comprenant (a) la culture d'une souche comprenant la séquence d'acides nucléiques mutante de la revendication 9 codant pour le polypeptide pour produire un surnageant comprenant le polypeptide ; et (b) la récupération du polypeptide.

11. Procédé de production de polypeptide selon l'une quelconque des revendications 1 à 2 comprenant (a) la culture d'une souche pour produire un surnageant comprenant le polypeptide ; et (b) la récupération du polypeptide.

12. Composition, en particulier une composition de détergent, comprenant un polypeptide selon l'une quelconque des revendications 1 à 2.

13. Utilisation du polypeptide selon l'une quelconque des revendications 1 à 2 dans une composition de détergent, en particulier une composition de détergent pour lessive et une composition de détergent pour vaisselle et des compositions de nettoyage pour revêtement en dur ou dans une composition de désencollage.
